(19) Europäisches Patentamt
European Patent Office
Office européen des brevets

(11) **EP 4 635 942 A1**

(12) **EUROPEAN PATENT APPLICATION**
published in accordance with Art. 153(4) EPC


(43) Date of publication:
**22.10.2025 Bulletin 2025/43**

(21) Application number: **23917211.7**

(22) Date of filing: **28.11.2023**

(51) International Patent Classification (IPC):
***C07D 215/38*** *(2006.01)* ***C07D 405/12*** *(2006.01)*
***C07D 401/12*** *(2006.01)* ***C07D 221/16*** *(2006.01)*
***A61P 29/00*** *(2006.01)* ***A61P 25/00*** *(2006.01)*
***A61P 35/00*** *(2006.01)* ***A61K 31/47*** *(2006.01)*
***A61K 31/473*** *(2006.01)*

(52) Cooperative Patent Classification (CPC):
**A61K 31/47; A61K 31/4709; A61K 31/473; A61K 31/497; A61K 31/506; A61K 31/706; A61P 1/00; A61P 9/10; A61P 11/00; A61P 11/06; A61P 17/00; A61P 17/06; A61P 19/02; A61P 19/08; A61P 25/00;** (Cont.)

(86) International application number:
**PCT/CN2023/134615**

(87) International publication number:
**WO 2024/152743 (25.07.2024 Gazette 2024/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **20.01.2023 CN 202310062976**



(71) Applicant: **Jiangsu Carephar Pharmaceutical Co., Ltd**
**Jiangsu 210000 (CN)**

(72) Inventors:
- **QIN, Yinlin**
**Nanjing, Jiangsu 210000 (CN)**
- **SU, Mei**
**Nanjing, Jiangsu 210000 (CN)**
- **WANG, Chaolei**
**Nanjing, Jiangsu 210000 (CN)**
- **ZHANG, Ya**
**Nanjing, Jiangsu 210000 (CN)**

(74) Representative: **Algemeen Octrooi- en Merkenbureau B.V.**
**P.O. Box 645**
**5600 AP Eindhoven (NL)**


(54) **QUINOLINE DERIVATIVE COMPOUNDS OR PHARMACEUTICALLY ACCEPTABLE SALTS THEREOF, PREPARATION METHOD THEREFOR AND USE THEREOF**


(57) Disclosed in the present invention are a quinoline derivative compounds or pharmaceutically acceptable salt thereof, preparation method therefor and use thereof. The structural formula of the quinoline derivative compounds provided by the present disclosure is shown in Formula I. The compounds or the pharmaceutically acceptable salts, and solvate or pharmaceutical composition thereof can be used to prepare a drug for preventing and/or treating inflammatory diseases. The quinoline derivative compounds provided by the present invention can achieve desired inhibitory effect on proinflammatory cytokines (such as IL-6, and TNF-$\alpha$) by upregulating miR-124, therefore said compounds can be used to treat inflammatory diseases such as ulcerative colitis.


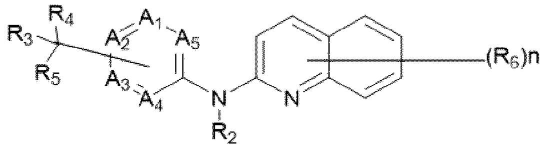

I

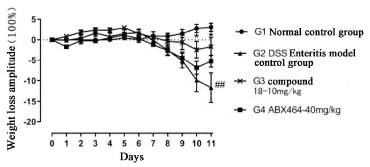


FIG. 1

EP 4 635 942 A1

(52) Cooperative Patent Classification (CPC): (Cont.)
**A61P 25/16; A61P 25/28; A61P 29/00;
A61P 35/00; A61P 37/06; C07D 215/38;
C07D 215/48; C07D 221/16; C07D 401/12;
C07D 405/12; C07H 1/00; C07H 17/02**

## Description

### CROSS REFERENCE

The present application claims priority to a prior application filed with the China National Intellectual Property Administration on January 20, 2023, with patent application number 202310062976.0, entitled "QUINOLINE DERIVATIVE **COMPOUNDS OR PHARMACEUTICALLY ACCEPTABLE SALT THEREOF, PREPARATION METHOD THEREFOR AND USE THEREOF".** The entire content of the above-mentioned prior application is incorporated herein by reference.

### TECHNICAL FIELD

The present application belongs to the technical field of small molecule medicines, and particularly relates to a quinoline derivative compounds or pharmaceutically acceptable salt thereof , preparation method therefor and use thereof.

### BACKGROUND OF THE INVENTION

Inflammation is a defensive response of the body to irritation and is closely related to the pathological process of many diseases. Related inflammatory diseases include autoimmune-related inflammatory diseases, inflammatory diseases in the central nervous system (CNS), inflammatory diseases in joints, inflammatory diseases in the digestive tract, inflammatory diseases in the skin, other inflammatory diseases related to epithelial cells, inflammation related to cancer, inflammation related to irritation, and inflammation related to injury.

Inflammatory bowel disease (IBD) is a chronic nonspecific intestinal inflammatory disease, typically including ulcerative colitis (UC) and Crohn's disease (CD). In recent years, with changes in residents' living standards and dietary habits, the incidence of IBDs has a rising tendency, with the incidence in developed countries being higher than that in developing countries. Among them, ulcerative colitis is a chronic disease that causes inflammation and ulcers in the colon and rectum. The main symptoms during the onset include abdominal pain, bloody diarrhea, weight loss, and fever. Complications of ulcerative colitis may include megacolon or inflammation of the eyes, joints or liver, as well as colon cancer.

Various cytokines are involved in immune response and inflammatory process, and they are typically divided into pro-inflammatory cytokines (such as IL-1, IL-6, and TNF-$\alpha$) and anti-inflammatory cytokines (such as IL-4, and IL-10). Studies have shown that the levels of pro-inflammatory cytokines (such as IL-6, and TNF-$\alpha$) in the intestinal mucosa of UC patients are elevated, and the secretion of anti-inflammatory cytokines is relatively insufficient, which causes excessive inflammatory response in the intestinal mucosa and causes intestinal damage. Among them, TNF-$\alpha$ is a key cytokine in the pathological process of IBDs. The serum level of TNF-$\alpha$ is related to the clinical activity of UC and Crohn's disease (CD). Anti-TNF-$\alpha$ antibodies are widely used in the treatment of IBDs and have achieved good results.

Studies have shown that miR-124 is specifically downregulated in pediatric patients with UC, which leads to an increase in expression of transducers and activators of transcription factor 3 (STAT3) and transcriptional activation of its downstream targets (such as proinflammatory cytokines IL-6, and TNF-$\alpha$) (Koukos et al.; Gastroenterology, 145(4):842-52:2013), and ultimately results in a series of inflammatory symptoms. Therefore, selectively upregulating miR-124 level in vivo play a role on inhibiting the expression of transducers and activators of transcription factor 3 (STAT3) and transcriptional activation of its downstream targets (such as proinflammatory cytokines IL-6, and TNF-$\alpha$).

Currently, the therapy of UC is a surgical or drug therapy. Commonly used therapeutic drugs include corticosteroids, aminosalicylic acid, glucocorticoids, antibiotics and immunomodulators. Sulfasalazine-related therapy is often effective for patients with mild UC, but has limited efficacy for moderate to severe patients. For patients with moderate to severe UC, corticosteroids are often used to rapidly induce disease mitigation. However, long-term use of steroids is not recommended for the treatment of the disease because of the potential for adverse reactions (e.g., osteoporosis and fractures, infection, cataracts, slower wound healing, and suppression of adrenal hormone production) related to long-term use. For patients with moderate to severe UC, systemic immunosuppressants such as azathioprine, cyclosporine, and methotrexate can reduce the frequency of episodes and have a certain degree of efficacy, but long-term use may be problematic since long-term systemic immunosuppression may have serious consequences (e.g., increased risk of infection and lymphoma). Anti-TNF-$\alpha$ antibodies (e.g., infliximab and adalimumab) are expensive and require subcutaneous or intravenous administration, and are only effective in approximately 60% to 70% of patients with moderate to severe UC. Moreover, approximately one-third of patients do not respond adequately, and another one-third of patients who initially respond gradually develop resistance after a few weeks of medication. The current mainstream UC therapy uses vedolizumab or anti-$\alpha 4\beta 7$ integrin antibodies, which are effective in patients with moderate to severe UC, but these drugs need to be injected, and many patients who receive the treatment do not get relief and eventually need a surgery.

Therefore, there is an urgent need to develop safer and more effective drugs with less toxic side effects.

The enzyme system most closely related to drug metabolism is the cytochrome CYP450 (CYP) enzyme system, which is typically present in the human liver and is also expressed in small amounts in the kidneys, small intestine, lungs, and brain. It participates in the biotransformation of many endogenous and exogenous substances (including most clinical drugs) in organisms. CYP is a gene superfamily that is involved in encoding more than 500 enzyme proteins. According to the similarity of proteins encoded by these genes, they are divided into different gene families and subfamilies. Among them, CYP1A2, CYP2C9, CYP2C19, CYP2D6, CYP2E1 and CYP3A4 are the six main subtypes of CYP450, accounting for about 80% of the total CYP450 enzymes in the liver. Among them, CYP3A4 accounts for 50% of the total CYP in the human body in most people, and 90% of drugs are metabolized by these six subtypes. Drug-drug interaction (DDI) should be considered in clinical medication, because there are many chemical substances that can enhance (inducer) or weaken (inhibitor) CYP activity, change the drug metabolism rate, and affect the effect of the drug. Among the identified drug interactions, drug interactions caused by the CYP450 enzyme system account for 70%. Therefore, understanding the type of drug catalyzed by each CYP is very meaningful for rational use of drugs in clinical practice, avoiding adverse drug reactions, implementing individualized dosing regimens, and demonstrating drug interactions that occur in the metabolic link.

Patent WO2015001518A1 discloses a compound ABX464, which is an oral small molecule used to treat ulcerative colitis by upregulating miR-124. It is currently in a Phase III clinical trial. However, it also has problems with drugability, such as unsatisfactory regulation of proinflammatory cytokines (such as IL-6, and TNF-$\alpha$) and strong inhibition of several subtypes of CYP450 enzymes.

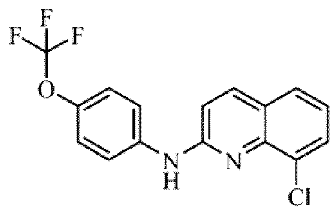

ABX-464

Therefore, finding compounds that have a more ideal regulatory effect on proinflammatory cytokines but without inhibition of CYP450 enzymes for use in the preparation of drugs for the treatment of inflammatory diseases is a problem that needs to be solved at present.

**SUMMARY OF THE INVENTION**

In order to solve the problem as mentioned in the technical background, the present application provides a quinoline derivative compound or pharmaceutically acceptable salt thereof , preparation method therefor and use thereof.

In a first aspect, the present application provides a quinoline derivative compound or pharmaceutically acceptable salt thereof, as shown in Formula I:

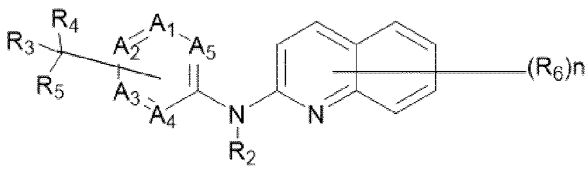

I

wherein,

$A_1$, $A_2$, $A_3$, $A_4$ and As are each independently selected from N or C-$R_1$, wherein $A_1$, $A_2$, $A_3$, $A_4$ and As comprise no more than 2 N;

$R_1$ is selected from hydrogen, deuterium, halogen, cyano, amino, hydroxyl, nitro, $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl, $C_1$-$C_3$ alkoxy and phosphate; wherein the $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, $C_1$-$C_3$ alkoxy are each independently substituted with one or more same or different substituents selected from $C_1$-$C_3$ alkyl, $C_1$-$C_6$ alkoxy, $C_3$-$C_7$ cycloalkyl, $C_3$-$C_6$ heterocycloalkyl, deuterium, halogen, cyano, amino, hydroxyl and nitro;

$R_2$ is selected from hydrogen, alkyl, cycloalkyl and heterocycloalkyl; wherein the alkyl, cycloalkyl and heterocycloalkyl are each independently substituted with one or more same or different substituents selected from halogen, hydroxyl, carboxyl, alkyl, alkoxy, deuterium, haloalkyl, haloalkoxy, nitro, amino and cyano;

$R_3$ and $R_4$ are each independently selected from cyano, hydroxyl, $C_1$-$C_3$ alkyl, $C_1$-$C_3$ alkanoyl, $C_1$-$C_3$ alkylamino, and $C_1$-$C_3$ alkoxy; wherein the $C_1$-$C_3$ alkyl, $C_1$-$C_3$ alkanoyl, $C_1$-$C_3$ alkylamino, and $C_1$-$C_3$ alkoxy are each independently substituted with one or more same or different substituents selected from halogen, hydroxyl, carboxyl, ester group, alkyl, alkoxy, deuterium, haloalkyl, haloalkoxy, nitro, amino and cyano;

or $R_3$ and $R_4$ are connected to form $C_3$-$C_6$ cycloalkyl or $C_3$-$C_6$ heterocycloalkyl;

$R_5$ is selected from halogen, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;

$R_3$, $R_4$ and $R_5$ are not all alkyl or cycloalkyl;

These $R_6$ are the same or different, and are each independently selected from hydrogen, deuterium, halogen, cyano, hydroxyl$C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl, phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro; and

n=1, 2 or 3.

Preferably, the structural formula of the quinoline derivative compound is shown in Formula I-1:

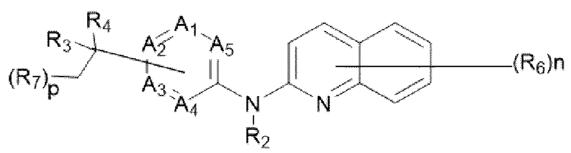

I-1

wherein:

these $R_7$ are the same or different, and are each independently selected from hydrogen, halogen, amino, hydroxyl, nitro, $C_1$-$C_3$ alkyl, $C_1$-$C_3$haloalkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl and $C_1$-$C_3$ alkoxy;

p=1, 2 or 3; and

$A_1$, $A_2$, $A_3$, $A_4$, $A_5$, $R_2$, $R_3$, $R_4$, and $R_6$ are as defined in claim 1.

Preferably, the structural formula of the quinoline derivative compound is shown in Formula I-2:

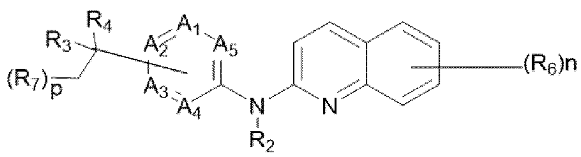

I-2.

Preferably, the $R_2$ is hydrogen or $C_3$-$C_8$ heterocycloalkyl; wherein the $C_3$-$C_8$ heterocycloalkyl is optionally substituted with one or more same or different substituents in hydroxyl and carboxyl.

Preferably, the structural formula of the quinoline derivative compound is shown in Formulae I-3, I-4 or 5:

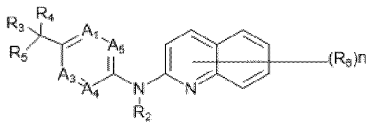

I-3

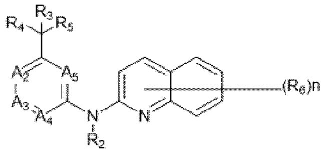

I-4

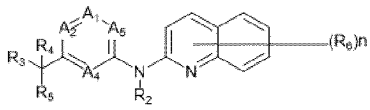

I-5

.

Preferably, the $R_3$ and $R_4$ are connected to form $C_3$-$C_6$ cycloalkyl or $C_3$-$C_6$ heterocycloalkyl, and together with $R_5$ to form a group of

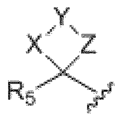

;

wherein, X, Y and Z are each independently selected from $(CH_2)_m$, NH or O; m=0, 1 or 2.

Preferably, the $R_3$ and $R_4$ are connected to form $C_3$-$C_6$ cycloalkyl or $C_3$-$C_6$ heterocycloalkyl, and together with $R_5$ to form a group as below:

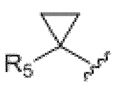 , 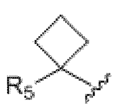 , 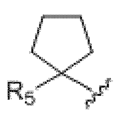 , 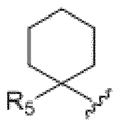 , 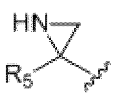 , 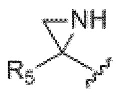 , 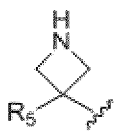 ,

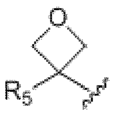 , 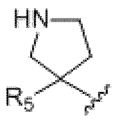 , 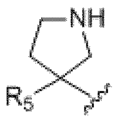 , 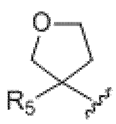 , 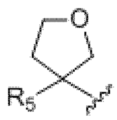 , 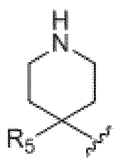 or 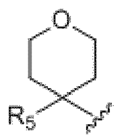 .

Preferably, the structural formula of the quinoline derivative compound is shown in Formulae I-7 or I-8:

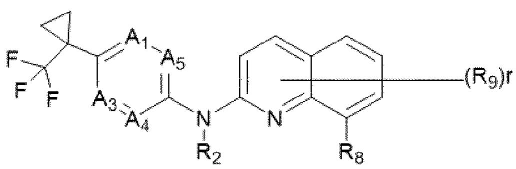

I-7

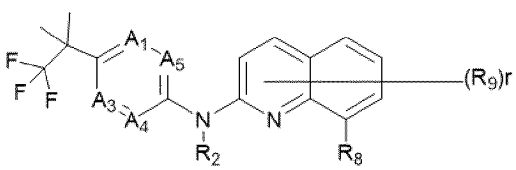

I-8

wherein:

$R_8$ is selected from deuterium, halogen, cyano, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;

these $R_9$ are the same or different, and are each independently selected from hydrogen, deuterium, halogen, cyano, hydroxyl, $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl, phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro; r=0, 1 or 2; and

$A_1$, $A_2$, $A_3$, $A_4$, $A_5$, $R_2$, $R_5$, and $R_6$ are as defined in claim 1.

Preferably, the $R_2$ is selected from hydrogen,

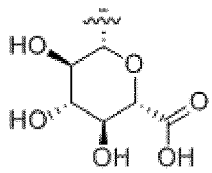

or

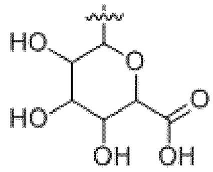

.

The quinoline derivative compound provided by the present application is preferably selected from any of the following compounds:

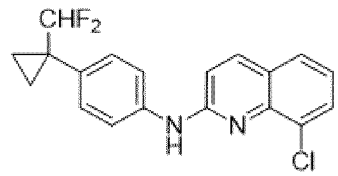

1

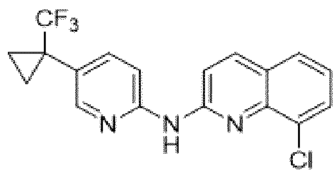

2

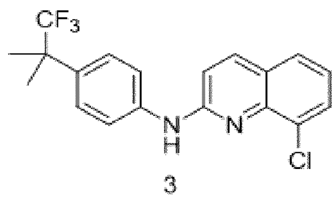

3

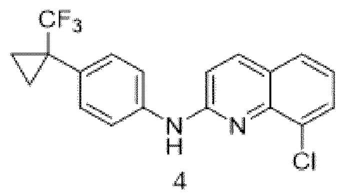

4

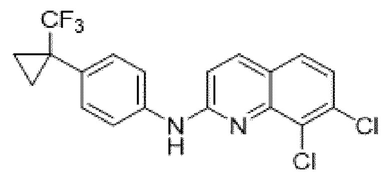

5

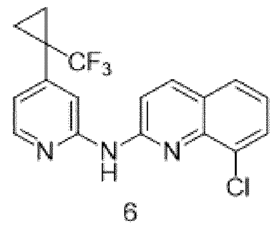

6

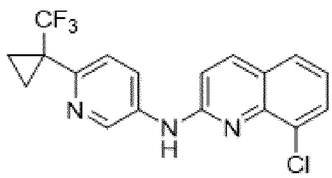

7

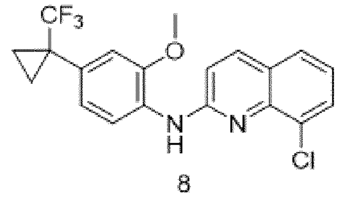

8

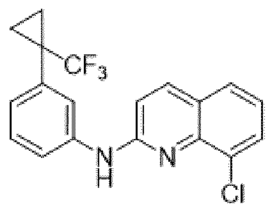

9

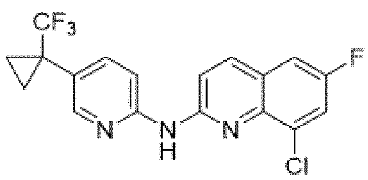

10

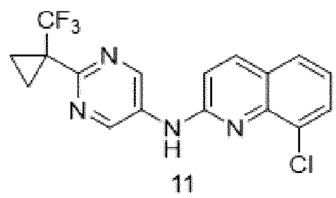

11

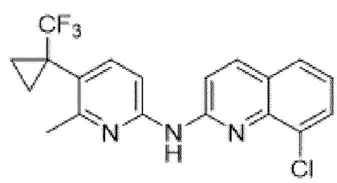

12

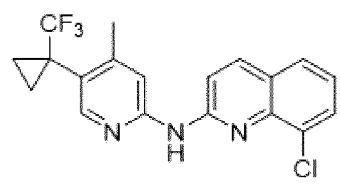
13

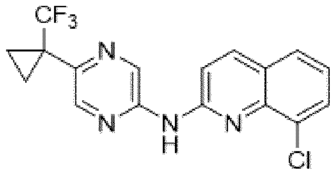
14

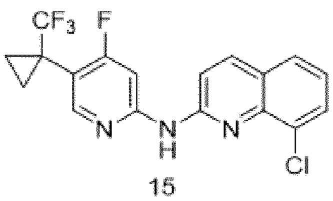
15

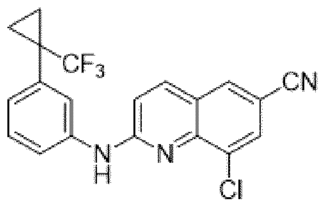
16

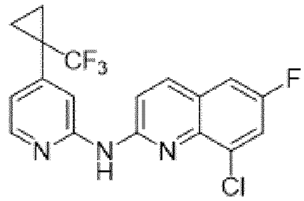
17

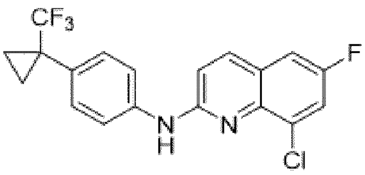
18

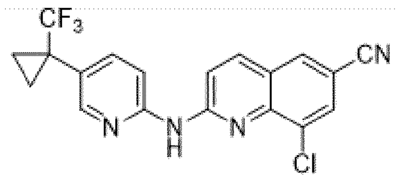
19

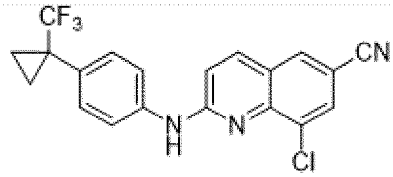
20

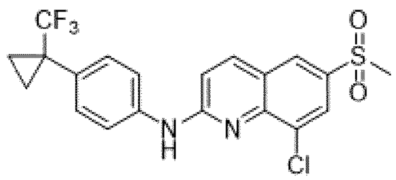
21

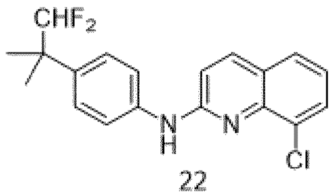
22

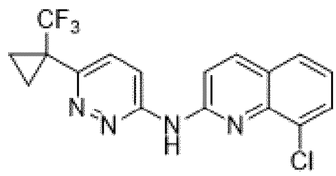
23

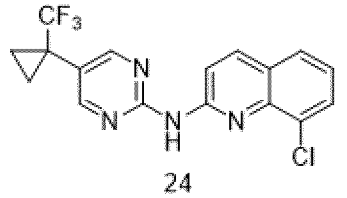
24

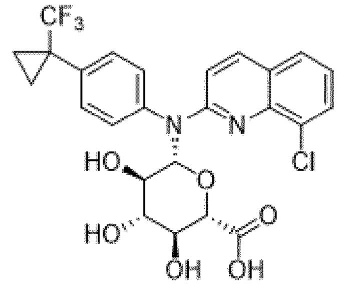
25

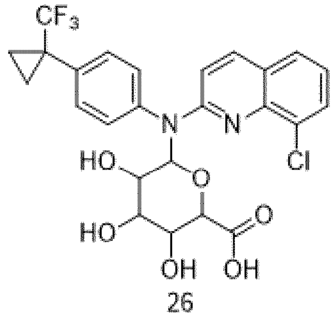
26

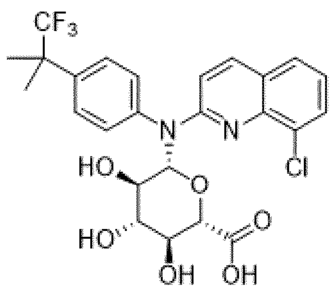
27

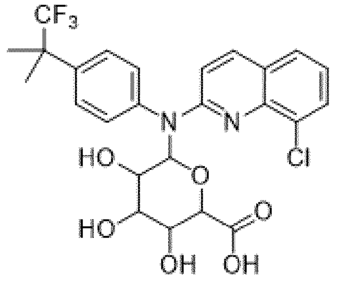
28

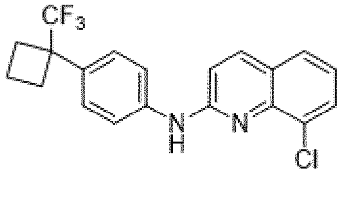
29

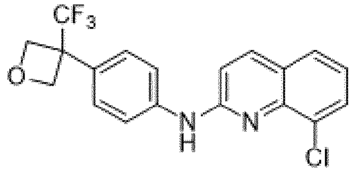
30

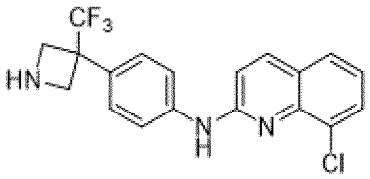

31

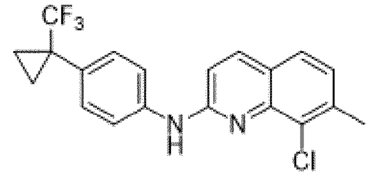

32

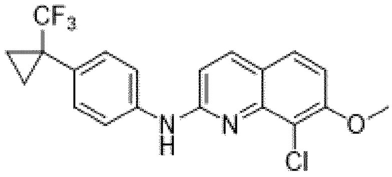

33

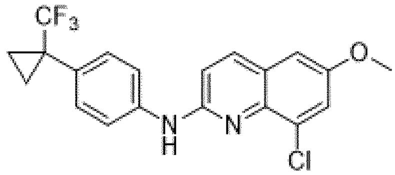

34

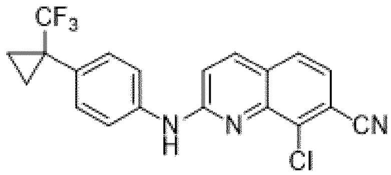

35

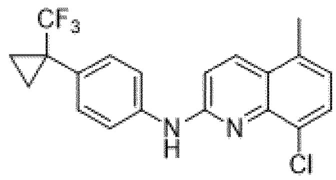

36

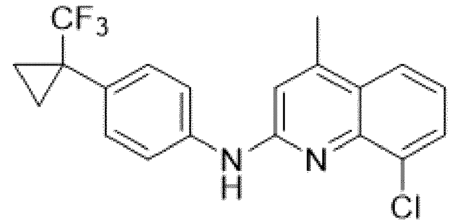

37

.

Secondly, the present application provides a method for preparing a quinoline derivative compound or a pharmaceutically acceptable salt thereof, which is used to prepare the above compound, comprising the following steps:

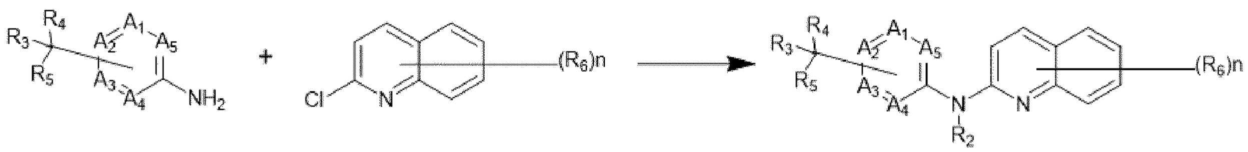

wherein:

$A_1$, $A_2$, $A_3$, $A_4$ and $A_5$ are each independently selected from N or C-$R_1$, wherein $A_1$, $A_2$, $A_3$, $A_4$ and As comprise no more than 2 N;

$R_1$ is selected from hydrogen, deuterium, halogen, cyano, amino, hydroxyl, nitro, $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl, $C_1$-$C_3$ alkoxy and phosphate; wherein the $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, $C_1$-$C_3$ alkoxy are each independently substituted with one or more same or different substituents selected from $C_1$-$C_3$ alkyl, $C_1$-$C_6$ alkoxy, $C_3$-$C_7$ cycloalkyl, $C_3$-$C_6$ heterocycloalkyl, deuterium, halogen, cyano, amino, hydroxyl and nitro;

$R_2$ is selected from hydrogen, alkyl, cycloalkyl and heterocycloalkyl; wherein the alkyl, cycloalkyl and heterocycloalkyl are each independently substituted with one or more same or different substituents selected from halogen, hydroxyl, carboxyl, alkyl, alkoxy, deuterium, haloalkyl, haloalkoxy, nitro, amino and cyano;

$R_3$ and $R_4$ are each independently selected from cyano, hydroxyl, $C_1$-$C_3$ alkyl, $C_1$-$C_3$ alkanoyl, $C_1$-$C_3$ alkylamino, and $C_1$-$C_3$ alkoxy;

or $R_3$ and $R_4$ are connected to form $C_3$-$C_6$ cycloalkyl or $C_3$-$C_6$ heterocycloalkyl;

$R_5$ is selected from deuterium, halogen, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;

$R_3$, $R_4$ and $R_5$ are not all alkyl or cycloalkyl;

these $R_6$ are the same or different, and are each independently selected from hydrogen, deuterium, halogen, cyano, hydroxyl$C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl,

phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro; and

n=1, 2 or 3.

Further, the present application provides a pharmaceutical composition comprising the above compound or a pharmaceutically acceptable salt thereof, and one or more pharmaceutically acceptable carriers, diluents or excipients.

Last, the present application also provides use of the above compound or a pharmaceutically acceptable salt thereof in the preparation of a drug for preventing and/or treating an inflammatory disease.

Further, the inflammatory disease is selected from autoimmune-related inflammatory diseases, inflammatory diseases in the central nervous system (CNS), inflammatory diseases in joints, inflammatory diseases in the digestive tract, inflammatory diseases in the skin, other inflammatory diseases related to epithelial cells, inflammation related to cancer, inflammation related to irritation, and inflammation related to injury.

Furthermore, the inflammatory disease is selected from inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and inflammation related to colon cancer.

Unless otherwise indicated, the following terms used in the specification and claims have the following meanings. A particular term should not be considered indefinite or unclear in the absence of a specific definition, but should be understood according to the common meaning in the art.

The term "pharmaceutically acceptable salt" refers to a salt of a pharmaceutically acceptable acid or base, including a salt formed by a compound with an inorganic acid or organic acid, and a salt formed by a compound with an inorganic base or an organic base.

The term "substituted" means that one or more hydrogen atoms in a given structure are replaced by a specific substituent. The one or more substituents may be different from each other or the same.

The term "optionally" or "optional" means that the event or environment described subsequently may but need not occur, and the description includes occasions where the event or environment occurs or does not occur. For example, "optionally halogen-substituted $C_1$-$C_6$ alkyl" means that halogen may but need not exist, and the description includes the case where the alkyl is substituted by halogen and the case where the alkyl is not substituted by halogen.

The term "deuterium" refers to deuteration, i.e., $^2$H or D.

The term "halogen" refers to fluorine, chlorine, bromine and iodine.

The term "hydroxy" refers to the -OH group.

The term "amino" refers to the $-NH_2$ group.

The term "cyano" refers to the -CN group.

The term "nitro" refers to the $-NO_2$ group.

The term "alkyl" refers to a saturated aliphatic hydrocarbon group, including straight and branched groups of 1 to 20 carbon atoms, preferably an alkyl group containing 1 to 6 carbon atoms, and more preferably an alkyl group containing 1 to 3 carbon atoms. The alkyl group may be straight or branched. For example, the term "$C_1$-$C_6$ alkyl" refers to an alkyl group containing 1, 2, 3, 4, 5 or 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, and 2-methylpentyl). Similarly, the alkyl moiety (i.e., alkyl) of alkoxy, alkanoyl, alkylphosphate, alkylsulfonyl and alkylaminosulfonyl has the same definition as above. For example, the term "$C_1$-$C_3$ alkyl" refers to an alkyl group containing 1, 2 or 3 carbon atoms (e.g., methyl, ethyl, propyl and isopropyl). The alkyl group may be substituted or unsubstituted. When substituted, the substituent may be located at any available attachment point, preferably being one or more of the following groups independently selected from deuterium, halogen, hydroxyl, nitro, amino, cyano, $C_1$-$C_6$ alkyl, $C_1$-$C_6$ alkoxy, $C_3$-$C_7$ cycloalkyl, 3-6 membered heterocycloalkyl, $C_6$-$C_{10}$ aryl and 5-10 membered heteroaryl, and $C_2$-$C_6$ alkenyl.

The term "alkoxy" refers to -O-alkyl. The term "$C_1$-$C_6$ alkoxy" may be understood as "$C_1$-$C_6$ alkyloxy" or "$C_1$-$C_6$ alkyl-O-"; it may be further preferably "$C_1$-$C_3$ alkoxy". Representative examples include, but are not limited to, methoxy, ethoxy, propoxy, butoxy, cyclopropyloxy, cyclobutyloxy, cyclopentyloxy, and cyclohexyloxy.

The term "alkylsulfonyl" refers to $-S(=O)_2$-alkyl.

The term "alkylacyl" refers to

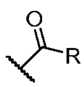

,

, where R is alkyl.

The term "ester group" refers to -C(=O)O-alkyl.

The term "$C_1$-$C_6$ alkanoyl" refers to the monovalent atomic group remaining after removing the hydroxyl group from the C1-C6 alkyl acid, which is usually also expressed as "$C_{0-5}$-C(=O)-". For example, "$C_1$-C(=O)-" refers to acetyl; "$C_2$-C(=O)-" refers to propionyl; "$C_3$-C(=O)-" refers to butyryl or isobutyryl.

The term "aminosulfonyl" refers to a group of formula $-S(=O)_2NH_2$.

The term "alkylamino" refers to a group in which an alkyl group is connected to a nitrogen atom, the nitrogen atom has at least one hydrogen atom, and the nitrogen atom can be connected to one alkyl group or two alkyl groups.

The term "$C_1$-$C_6$ alkylaminosulfonyl" refers to a group of formula $-S(=O)_2NH$(alkyl), in which the alkyl group has 1 to 6 carbon atoms. In some embodiments, the alkyl group has 1 to 6, 1 to 4, or 1 to 3 carbon atoms.

The term "phosphate" refers to $-PO_3H$.

The term "$C_1$-$C_6$ alkylphosphate" refers to the group $-PO_3$(alkyl).

The term "haloalkyl" refers to an alkyl group substituted with one or more halogens, in which the alkyl group is as defined above.

The term "haloalkoxy" refers to an alkoxy group substituted with one or more halogens, in which the alkoxy group is as defined above.

The term "$C_2$-$C_6$ alkenyl" refers to a linear or branched monovalent hydrocarbon group containing 2-6 carbon atoms (e.g., 2, 3, 4, 5, and 6 carbon atoms). Specific examples include but are not limited to vinyl ($-CH=CH_2$), allyl ($-CH_2CH=CH_2$), and the like.

The term "$C_2$-$C_6$ alkynyl" refers to a group containing 2-6 carbon atoms (e.g., 2, 3, 4, 5, and 6 carbon atoms) and one or two triple bonds, which may be, but is not limited to, ethynyl, propargyl, butynyl, isobutynyl, pentynyl, isopentenyl, and hexynyl.

The term "cycloalkyl" refers to a fully saturated carbon ring that exists in the form of a monocyclic, cyclic, bridged, or spirocyclic ring. Unless otherwise indicated, the carbon ring is typically a 3- to 10-membered ring. Preferably, it contains 3 to 8 ring atoms. Specific examples of cycloalkyl include, but are not limited to, cyclopropyl, cyclobutyl, cyclopentyl, cyclohexyl, cycloheptyl, cyclooctyl, cyclononyl, cyclodecyl, norbornyl (bicyclo[2.2.1]heptyl), bicyclo[2.2.2]octyl, adamantyl, and spiro[4.5]decane. Spirocycloalkyl refers to a cycloalkyl group that exists in a spirocyclic ring. The term "$C_3$-$C_7$ cycloalkyl" should be understood to mean a saturated monovalent monocyclic, cyclic, spirocyclic or bridged ring having 3 to 7 carbon atoms, specific examples including but not limited to cyclopropyl, cyclobutyl, cyclopentyl, and cyclohexyl. The cycloalkyl group may be substituted or unsubstituted. When substituted, the substituents are preferably one or more, more preferably one, two or three, and even more preferably one or two, and the substituents are selected from halogen, hydroxyl, nitro, amino, cyano, $C_1$-$C_6$ alkyl, $C_1$-$C_6$ alkoxy, $C_3$-$C_6$ cycloalkyl, 3 to 6 membered heterocycloalkyl, $C_6$-$C_{10}$ aryl, 5 to 10 membered heteroaryl, and $C_2$-$C_6$ alkenyl.

The term "heterocycloalkyl" refers to a saturated or partially unsaturated cyclic group in the form of a monocyclic, cyclic, bridged or spirocyclic ring, containing 3 to 20 ring atoms, in which the ring atoms of the heterocyclic ring contain 1-5 heteroatoms, including but not limited to nitrogen (N), oxygen (O), sulfur (S), phosphorus (P), $-S(=O)_2-$, and -S(=O)-, and the remaining ring atoms are carbon. Preferably, it contains 3 to 12 ring atoms, of which 1 to 4 are heteroatoms; more preferably, it contains 3 to 8 ring atoms, and more preferably, it contains 3 to 6 ring atoms. The term "$C_3$-$C_6$ heterocycloalkyl" refers to a group containing 3 to 6 ring atoms.

Examples of 3-membered heterocycloalkyl include but are not limited to oxiranyl, thiiranyl, and aziridinyl. Examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiopyranyl, morpholinyl, piperazinyl, 1,4-thioxanyl, 1,4-dioxanyl, thiomorpholinyl, 1,3-dithianyl, and 1,4-dithianyl. The heterocycloalkyl may be substituted or unsubstituted. When substituted, the substituents are preferably one or more, more preferably one, two or three, and even more preferably one or two, and the substituents are selected from halogen, hydroxyl, carboxyl, nitro, amino, cyano, $C_1$-$C_6$ alkyl, $C_1$-$C_6$ alkoxy, $C_3$-$C_6$ cycloalkyl, 3-6 membered heterocycloalkyl, $C_6$-$C_{10}$ aryl, 5-10 membered heteroaryl, and $C_2$-$C_6$ alkenyl.

The term "heteroaryl" is a monocyclic, bicyclic or tricyclic ring system containing one, two or three aromatic rings and at least one nitrogen, oxygen or sulfur atom in the aromatic ring, and it may be unsubstituted or substituted. The heteroaryl group may be any divalent group. Examples of heteroaryl groups include, but are not limited to, 2H-pyrrolyl, 3H-indolyl, 4H-quinolizinyl, 4H-carbazolyl, acridinyl, benzo[b]thienyl, benzothiazolyl, β-carbolinyl, carbazolyl, chromenyl, cinnaolinyl, dibenzo[b,d]furanyl, furazolidinyl, furanyl, imidazolyl, imidizolyl, indazolyl, indolisinyl, indolyl, isobenzofuranyl, isoindolyl, isoquinolinyl, isothiazolyl, isoxazolyl, naphthyridinyl, naphthy[2,3-b], and oxazolyl.

"Pharmaceutically acceptable carrier" refers to inactive ingredients in a pharmaceutical composition that do not cause significant irritation to an organism and do not interfere with the biological activity and properties of the compound to be administered, including but not limited to any glidant, sweetener, diluent, preservative, dye/colorant, flavor enhancer,

surfactant, wetting agent, dispersant, disintegrant, suspending agent, stabilizer, isotonic agent, solvent or emulsifier.

The term "treatment" or "treating" means administering the compound or preparation described in the present application to improve or eliminate a disease or one or more symptoms associated with the disease, and includes: (i) inhibiting a disease or disease state, that is, curbing its development; and (ii) alleviating a disease or disease state, that is, the disease or disease state subsides.

The term "prevention" or "prevent" means administering the compound or preparation described in the present application to prevent a disease or one or more symptoms associated with the disease, including: preventing a disease or disease state from occurring in a mammal, especially when such mammal is susceptible to the disease state but has not yet been diagnosed as having the disease state.

The term "therapeutically effective amount" means that when administered to a subject to treat a disease, the amount of the compound is sufficient to be effective in treating the disease. The "therapeutically effective amount" may vary depending on the compound, the disease and its severity, and the condition, age, weight, sex, etc. of the subject to be treated.

The term "pharmaceutical composition" refers to a mixture of one or more compounds of the present application or salts thereof and a pharmaceutically acceptable carrier. The purpose of a pharmaceutical composition is to facilitate the administration of the compounds of the present application to an organism.

## BRIEF DESCRIPTION OF THE DRAWINGS

Figure 1 shows the weight change of mice in each group during the drug efficacy period;
Figure 2 shows the DAI score change of mice in each group during the drug efficacy period.

## DETAILED DESCRIPTION OF THE INVENTION

The following will be combined with the drawings in the examples of the present application to clearly and completely describe the technical solutions in the examples of the present invention. Apparently, the described examples are only part of, not all of, the examples of the present invention. Based on the examples of the present invention, all other examples obtained by ordinary skilled in the art without any creative efforts are within the scope of protection of the present invention.

Example 1. 8-chloro-N-(4-(1-(difluoromethyl)cyclopropyl)phenyl)quinolin-2-amine (Compound 1)

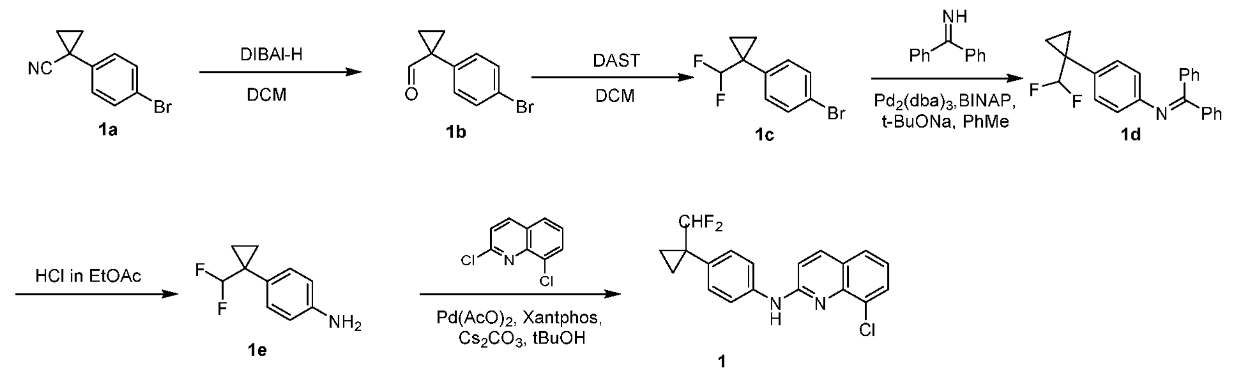


1a 1b 1c 1d 1e 1

Step 1: 1-(4-bromophenyl)cyclopropan-1-carboxaldehyde

At -78°C, under nitrogen protection, 1-(4-bromophenyl)cyclopropan-1-carbonitrile (1a) (0.50 g, 2.25 mmol) was dissolved in ultra-dry dichloromethane solution (12 mL), and DIBAL-H (1M, 2.7 mL, 2.70 mmol) was added dropwise. After the dropwise addition was completed, the mixture was stirred at -78°C for 2 hours and then warmed to room temperature and stirred for 2 hours to terminate the reaction. Under ice cooling, 1M aqueous hydrochloric acid solution (6 mL) was added to quench the reaction. Dichloromethane was added for extraction, and the resultant was washed, dried, filtered and concentrated. The crude product was purified by silica gel column chromatography (eluent, petroleum ether: ethyl acetate = 6: 1) to obtain the target compound of 1-(4-bromophenyl)cyclopropan-1-carboxaldehyde (1b).

$^1H$ NMR (400 MHz, $CDCl_3$) δ 9.15 (s, 1H), 7.52-7.45 (m, 2H), 7.22-7.15 (m, 2H), 1.60-1.55 (m, 2H), 1.41-1.36 (m, 2H).

Step 2: 1-bromo-4-(1-(difluoromethyl)cyclopropyl)benzene

At room temperature, 1-(4-bromophenyl)cyclopropane-1-carbaldehyde (1b) (0.40 g, 1.60 mmol) was dissolved in dichloromethane (12 mL). The mixture was cooled to -20°C and DAST (0.65 g, 4.00 mmol) was added dropwise and stirred for 10 minutes. The reaction was carried out by warming to room temperature for 3 hours until terminated. The reaction was quenched by adding water. The mixture was adjusted to pH 8-9 with saturated sodium bicarbonate solution. The resultant was extracted with ethyl acetate, washed, dried, filtered, and concentrated. The crude product was purified by silica gel column chromatography (eluent, petroleum ether: ethyl acetate = 1: 0) to obtain the target compound of 1-bromo-4-(1-(difluoromethyl)cyclopropyl)benzene (1c).

$^1H$ NMR (400 MHz, $CDCl_3$) δ 7.46 (d, *J* = 8.4 Hz, 2H), 7.28 (d, *J* = 8.4 Hz, 2H), 5.58 (t, *J*= 57.0 Hz, 1H), 1.15 (q, *J* = 5.0 Hz, 2H), 0.97 - 0.91 (m, 2H).

Step 3: N-(4-(1-(difluoromethyl)cyclopropyl)phenyl)-1,1-diphenylmethaneimine

At room temperature, under nitrogen protection, 1-Bromo-4-(1-(difluoromethyl)cyclopropyl)benzene (1c) (0.20 g, 0.81 mmol), benzophenone imine (0.18 g, 0.97 mmol), $Pd_2(dba)_3$ (14.83 mg, 0.03 mmol), BINAP (30.24 mg, 0.05 mmol) and sodium tert-butoxide (0.12 g, 1.21 mmol) were added to a toluene solution (10 mL). The reaction was carried out by stirring at 90 °C for 10 h under a nitrogen atmosphere until terminated. After cooling to room temperature, the reaction solution was filtered through celite and the filtrate was concentrated in vacuo. The crude product was purified by silica gel column chromatography (eluent, petroleum ether: ethyl acetate = 5: 1) to obtain the target compound N-(4-(1-(difluoromethyl)cyclopropyl)phenyl)-1,1-diphenylmethaneimine (1d).

LCMS (ESI) calcd for $C_{23}H_{19}F_2N$ $[M + H]^+$ m/z 348, found 348.1.

Step 4: 4-(1-(difluoromethyl)cyclopropyl)aniline

At room temperature, N-(4-(1-(difluoromethyl)cyclopropyl)phenyl)-1,1-diphenylmethaneimine (1d) (0.20 g, 0.58 mmol) was dissolved in ethyl acetate (3 mL), and hydrochloric acid/ethyl acetate solution (4 M, 6 mL) was added. The reaction was carried out at room temperature for 2 hours until terminated. The mixture was added with saturated sodium carbonate solution to adjust the pH to 11, extracted with ethyl acetate, dried, filtered and concentrated. The crude product was purified by silica gel column chromatography (eluent, petroleum ether: ethyl acetate = 5: 1) to obtain the target compound of 4-(1-(difluoromethyl)cyclopropyl)aniline (1e).

LCMS (ESI) calcd for $C_{10}H_{11}F_2N$ $[M + H]^+$ m/z 184, found 184.1.

Step 5: 8-chloro-N-(4-(1-(difluoromethyl)cyclopropyl)phenyl)quinolin-2-amine

At room temperature, under nitrogen protection, 4-(1-(difluoromethyl)cyclopropyl)aniline (1e) (0.10 g, 0.55 mmol), 2, 8-dichloroquinoline (0.13 g, 0.66 mmol), palladium acetate (6.13 mg, 0.03 mmol), Xantphos (15.79 mg, 0.03 mmol) and cesium carbonate (0.53 g, 1.64 mmol) were added to tert-butyl alcohol solution (50 mL). The mixture was stirred at 90 °C for 3 h under a nitrogen atmosphere to carried out the reaction until terminated. After cooling to room temperature, the reaction solution was filtered through celite and the filtrate was concentrated in vacuo. The crude product was purified by silica gel column chromatography (eluent, petroleum ether: ethyl acetate = 4: 1) to obtain the target compound of 8-chloro-N-(4-(1-(difluoromethyl)cyclopropyl)phenyl)quinolin-2-amine(Compound 1).

LCMS (ESI) calcd for $C_{19}H_{15}ClF_2N_2$ $[M + H]^+$ m/z 345, found 345.1.

$^1H$ NMR (400 MHz, DMSO) δ 9.70 (s, 1H), 8.13 (dd, *J* = 12.4, 8.8 Hz, 3H), 7.77 (dd, *J* = 7.6, 1.2 Hz, 1H), 7.72 (dd, *J* = 8.0, 1.2 Hz, 1H), 7.35 (d, *J* = 8.6 Hz, 2H), 7.27 (t, *J* = 7.8 Hz, 1H), 7.14 (d, *J* = 9.2 Hz, 1H), 5.85 (t, *J* = 56.8 Hz, 1H), 1.09 (dd, *J* = 6.4 Hz, 4.8 Hz, 2H), 0.99 - 0.91 (m, 2H).

Example 2. 8-chloro-N-(5-(1-(trifluoromethyl)cyclopropyl)pyridin-2-yl)quinolin-2-amine (Compound 2)

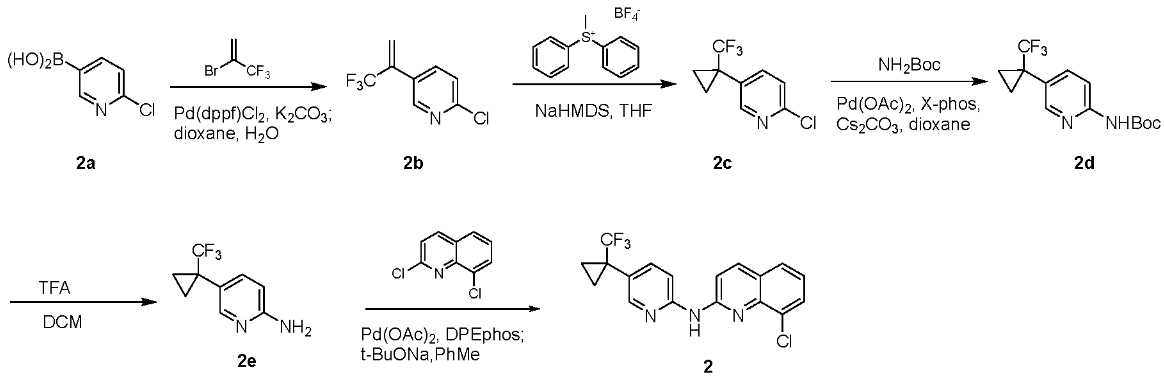


2a 2b 2c 2d 2e 2

Step 1: 2-chloro-5-(3,3,3-trifluoropropyl-1-en-2-yl)pyridine

At room temperature, under nitrogen protection, (6-chloropyridin-3-yl)boronic acid (2a) (5.00 g, 31.77 mmol), 2-bromo-3,3,3-trifluoroprop-1-ene (8.39 g, 47.96 mmol), Pd(dppf)$Cl_2$ (2.33 g, 3.18 mmol) and potassium carbonate (10.09 g, 79.52 mmol) were added to 1,4-dioxane:water (5:1; 30 mL). The mixture was stirred at 70 °C for 6 h under a nitrogen atmosphere to carry out the reaction until terminated. After cooling to room temperature, the reaction solution was filtered through celite, extracted with ethyl acetate, dried, filtered, and concentrated. The crude product was purified by silica gel column chromatography (eluent, petroleum ether: ethyl acetate = 12: 1) to obtain the target compound of 2-chloro-5-(3,3,3-trifluoropropyl-1-en-2-yl)pyridine (2b).

$^1$H NMR (400 MHz, DMSO-$d_6$) $\delta$ 8.56 (d, *J* = 2.4Hz, 1H), 7.98 (dd, *J* = 8.4, 2.2Hz, 1H), 7.64 (d, *J* = 8.4Hz, 1H), 6.34-6.30 (m, 1H), 6.28-6.23 (m, 1H).

Step 2: 2-chloro-5-(1-(trifluoromethyl)cyclopropyl)pyridine

At room temperature, under nitrogen protection, 2-chloro-5-(3,3,3-trifluoropropyl-1-en-2-yl)pyridine (2b) (1.20 g, 5.75 mmol) and diphenyl(methyl)sulfonium tetrafluoroborate (2.15 g, 7.48 mmol) were dissolved in ultra-dry tetrahydrofuran (15 ml), cooled to 0°C, and NaHMDS (1 M in THF, 9.2 mL, 9.20 mmol) was slowly added dropwise. The solution was reacted at 0°C for 15 minutes. The reaction was carried out by warming to room temperature for 3 hours until terminated. The mixture was quenched by adding saturated ammonium chloride solution, extracted with ethyl acetate, dried, filtered, and concentrated. The crude product was purified by silica gel column chromatography (eluent, petroleum ether: ethyl acetate=15: 1) to obtain the target compound of 2-chloro-5-(1-(trifluoromethyl)cyclopropyl)pyridine(2c).

$^1$H NMR (400 MHz, DMSO-$d_6$) $\delta$ 8.51 (d, *J* = 2.4 Hz, 1H), 7.96 (dd, *J* = 8.0, 2.4 Hz, 1H), 7.57 (dd, *J* = 8.4 Hz, 0.4 Hz, 1H), 1.40 (dd, *J* = 7.2, 5.2 Hz, 2H), 1.26 - 1.20 (m, 2H).

Step 3: Tert-butyl (5-(1-(trifluoromethyl)cyclopropyl)pyridin-2-yl)carbamate

At room temperature, under nitrogen protection, 2-chloro-5-[1-(trifluoromethyl)cyclopropyl]pyridine (2c) (0.50 g, 2.26 mmol), tert-butyl carbamate (1.06 g, 9.02 mmol), palladium acetate (25.33 mg, 0.11 mmol), Xphos (0.11 g, 0.23 mmol) and cesium carbonate (2.21 g, 6.77 mmol) were added to a 1,4-dioxane solution (12 mL). The mixture was stirred at 90 °C for 4 h under a nitrogen atmosphere to carry out the reaction until terminated. After cooling to room temperature, the reaction solution was filtered through celite. The resultant was extracted with ethyl acetate, washed, dried, filtered, and concentrated. The crude product was purified by silica gel column chromatography (eluent, petroleum ether: ethyl acetate = 4: 1) to obtain the target compound of Tert-butyl (5-(1-(trifluoromethyl)cyclopropyl)pyridin-2-yl)carbamate (2d).

LCMS (ESI) calcd for $C_{14}H_{17}F_3N_2O_2$ $[M+H]^+$ m/z 303, found 302.9.

Step 4: 5-(1-(trifluoromethyl)cyclopropyl)pyridine-2-amine

At room temperature, tert-butyl (5-(1-(trifluoromethyl)cyclopropyl)pyridin-2-yl)carbamate (2d) (0.11 g, 0.36 mmol) was dissolved in dichloromethane (10 mL), and trifluoroacetic acid (2 mL) was added. The reaction was carried out at room temperature for 2 hours until terminated. The mixture was adjusted to pH 9 with saturated sodium carbonate solution, extracted with dichloromethane, washed, dried, and filtered to obtain the target compound of 5-(1-(trifluoromethyl)cyclopropyl)pyridine-2-amine (2e).

LCMS (ESI) calcd for $C_9H_9F_3N_2$ $[M+H]^+$ m/z 203, found 203.1.

Step 5: 8-chloro-N-(5-(1-(trifluoromethyl)cyclopropyl)pyridin-2-yl)quinolin-2-amine

The preparation process of Example 1 Step 5 was followed to prepare the target compound of 8-chloro-N-(5-(1-(trifluoromethyl)cyclopropyl)pyridin-2-yl)quinolin-2-amine (Compound 2).

LCMS (ESI) calcd for $C_{18}H_{13}ClF_3N_3$ $[M+H]^+$ m/z 364, found 363.7.

$^1H$ NMR (400 MHz, DMSO-$d_6$) δ 10.47 (s, 1H), 9.02 (d, *J* = 8.8Hz, 1H), 8.39 (d, *J* = 2.2Hz, 1H), 8.23 (d, *J* = 9.0Hz, 1H), 7.91 (dd, *J* = 8.4, 2.0Hz, 1H), 7.84-7.78 (m, 2H), 7.49 (d, *J = 8.8Hz,* 1H), 7.35 (t, *J* = 7.8Hz,1H), 1.36 (dd, *J* = 6.6, 5.2Hz, 2H), 1.31-1.22 (m, 2H).

Example 3. 8-chloro-N-(4-(1,1,1-trifluoro-2-methylprop-2-yl)phenyl)quinolin-2-amine (Compound 3)

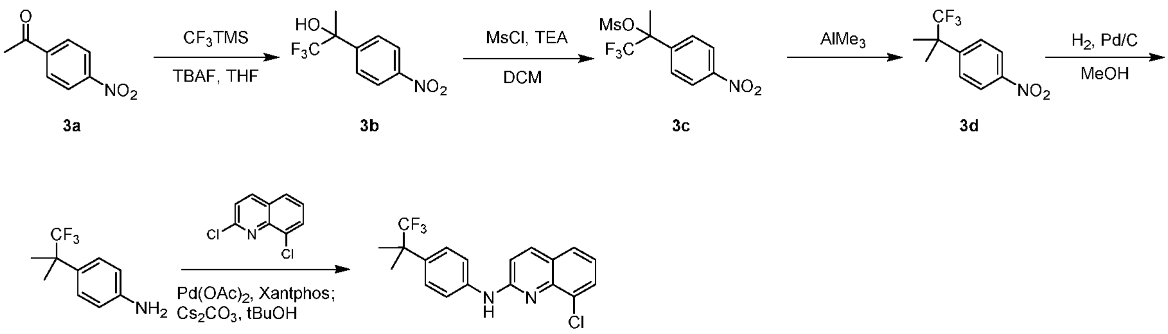

3a 3b 3c 3d
3e 3

Step 1: 1,1,1-trifluoro-2-(4-nitrophenyl)propan-2-ol

At 0°C, under nitrogen protection, 1-(4-nitrophenyl)ethanone (3a) (5.00 g, 30.00 mmol), TMS-CF3 (6.46 g, 45.00 mmol), and TBAF (15 mL, 15.00 mmol) were added to a THF (50 mL) solution. The reaction was carried out by stirring at room temperature for 1 hour until terminated. The mixture was quenched with water and extracted with ethyl acetate. The combined organic phases were washed with water, dried and concentrated. The crude product was purified by silica gel column chromatography (eluent, petroleum ether: ethyl acetate:5:1), to obtain the target compound of 1,1,1-trifluoro-2-(4-nitrophenyl)propan-2-ol (3b).

$^1H$ NMR (400 MHz, $CDCl_3$) δ 8.25 (d, J = 8.8Hz, 2H), 7.79 (d, J = 8.8Hz, 2H), 2.80 (s, 1H), 1.83 (s, 3H).

Step 2: 1,1,1-trifluoro-2-(4-nitrophenyl)prop-2-yl methanesulfonate

At 0°C, under nitrogen protection, MsCl (0.58 g, 5.00 mmol) was slowly added dropwise to a solution of 1,1,1-trifluoro-2-(4-nitrophenyl)propan-2-ol (3b) (1.00 g, 4.00 mmol) and triethylamine (1.27 g, 12.60 mmol) in DCM (10 mL). After the dropwise addition was complete, the reaction solution was stirred at room temperature to carry out the reaction for 2 hours until terminated. The mixture was quenched with water, extracted with DCM, washed with water, dried and concentrated. The crude product was separated and purified by silica gel column chromatography (eluent, petroleum ether: ethyl acetate:5:1) to obtain the target compound of 1,1,1-trifluoro-2-(4-nitrophenyl)prop-2-yl methanesulfonate (3c).

1H NMR (400 MHz, $CDCl_3$) δ 8.33-8.27 (m, 2H), 7.77 (d, J = 8.8Hz, 2H), 3.21 (s, 3H), 2.34(d, J = 0.8 Hz, 3H).

Step 3: 1-nitro-4-(1,1,1-trifluoro-2-methyl-2-propyl)benzene

At 0°C, under nitrogen protection, 1,1,1-trifluoro-2-(4-nitrophenyl)propan-2-yl methanesulfonate (3c) (0.50 g, 1.59 mmol) and trimethylaluminum (2 M in Hexanes, 1.6 mL, 3.18 mmol) were added together to the DCM (10 mL) solution. The reaction was carried out by stirring at room temperature for 2 hours until terminated. The mixture was quenched with water, extracted with DCM, washed with water, dried and concentrated. The crude product was separated and purified by silica gel column chromatography (eluent, petroleum ether: ethyl acetate:5:1) to obtain the target compound of 1-nitro-4-(1,1,1-trifluoro-2-methyl-2-propyl)benzene (3d).

$^1H$ NMR (400 MHz, $CDCl_3$) δ 8.22 (d, J = 8.6Hz, 2H), 7.68 (d, J = 8.8Hz, 2H), 1.63(s, 6H).

Step 4: 4-(1,1,1-trifluoro-2-methyl-2-methylpropyl)aniline

At room temperature, under hydrogen atmosphere, 1-nitro-4-(1,1,1-trifluoro-2-methyl-2-propyl)benzene (3d) (0.13 g, 0.56 mmol) and palladium/carbon (0.06 g, 1.15 mmol) were added together to a solution of methanol (3 mL) and THF (1 mL). The mixture was stirred at room temperature to carry out the reaction for 2 hours until terminated. The reaction solution was filtered through celite and concentrated. The crude product was purified by silica gel column chromatography (eluent, petroleum ether:ethyl acetate:5:1), to obtain the target compound of 4-(1,1,1-trifluoro-2-methyl-2-methylpropyl) aniline (3e).

1H NMR (400 MHz, DMSO-d6) δ 7.14 (d, J = 8.4Hz, 2H), 6.54 (d, J = 8.6Hz, 2H), 5.10 (s, 2H), 1.45 (s, 6H).

Step 5: 8-chloro-N-(4-(1,1,1-trifluoro-2-methylprop-2-yl)phenyl)quinolin-2-amine

The preparation process of Example 1 Step 5 was followed to prepare the target compound of 8-chloro-N-(4-(1,1,1-trifluoro-2-methylprop-2-yl)phenyl)quinolin-2-amine (Compound 3).

LCMS (ESI) calcd for C19H16ClF3N [M+H]+ 365.10 m/z, found 365.00.

1H NMR (400 MHz, DMSO-d6) δ 9.79 (s, 1H), 8.20 (d, J = 8.8Hz,2H), 8.13 (d, J = 8.8Hz, 1H), 7.76 (dd, J = 16.4, 7.8Hz, 2H), 7.50 (d, J = 8.6Hz, 2H), 7.28 (t, J = 7.8Hz, 1H), 7.15 (d, J = 8.8Hz, 1H), 1.56 (s, 6H).

Example 4. 8-chloro-N-(4-(1-(trifluoromethyl)cyclopropyl)phenyl)quinolin-2-amine (Compound 4)

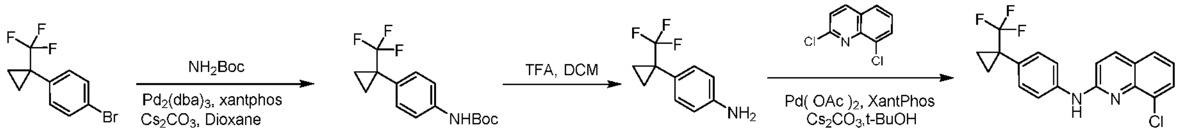


4a 4b 4c 4

Step 1: Tert-Butyl (4-(1-(trifluoromethyl)cyclopropyl)phenyl)carbamate

At room temperature, under nitrogen protection, 1-bromo-4-(1-(trifluoromethyl)cyclopropyl)benzene (4a) (0.84 g, 3.17 mmol), tert-butyl carbamate (1.48 g, 12.68 mmol), $Pd_2(dba)_3$ (0.29 g, 0.32 mmol), Xantphos (0.55 g, 0.96 mmol) and cesium carbonate (5.16 g, 15.85 mmol) were added to a 1,4-dioxane solution (30 mL). The mixture was stirred at 115 °C to carry out the reaction for 7 hours under a nitrogen atmosphere until terminated. After cooling to room temperature, the reaction solution was filtered through celite and the filtrate was concentrated in vacuo. The crude product was purified by silica gel column chromatography (eluent, petroleum ether: ethyl acetate=15: 1) to obtain tert-Butyl (4-(1-(trifluoromethyl) cyclopropyl)phenyl)carbamate (4b).

$^1$H NMR (400 MHz, $CDCl_3$) δ7.37 (d, *J* =8.6Hz, 2H), 7.32 (d, *J* =8.6Hz, 2H), 6.48 (s, 1H), 1.51 (s, 9H), 1.51-1.30 (m, 2H), 1.04-0.88 (m, 2H).

Step 2: 4-(1-(trifluoromethyl)cyclopropyl)aniline

At room temperature, tert-butyl (4-(1-(trifluoromethyl)cyclopropyl)phenyl)carbamate (4b) (0.50 g, 1.66 mmol) was dissolved in dichloromethane (4 mL), and trifluoroacetic acid (4 mL) was added. The mixed solution was stirred at 25°C to carry out the reaction for 1 hour until terminated. After the reaction was completed, the mixture was adjusted to pH 11 with saturated sodium bicarbonate solution and extracted with dichloromethane solution (3×10 mL). The combined organic phases were washed with water and brine, dried over anhydrous sodium sulfate, filtered, and the filtrate was collected. The filtrate was concentrated in vacuo to obtain the crude product of 4-(1-(trifluoromethyl)cyclopropyl)aniline (4c).

LCMS(ESI) calcd for $C_{10}H_{10}F_3N$ $[M+H]^+$ m/z 202, found 202.1.

Step 3: 8-chloro-N-(4-(1-(trifluoromethyl)cyclopropyl)phenyl)quinolin-2-amine

The preparation process of Example 1 Step 5 was followed to prepare the target compound of 8-chloro-N-(4-(1-(trifluoromethyl)cyclopropyl)phenyl)quinolin-2-amine (Compound 4).

LCMS(ESI) calcd for $C_{19}H_{14}ClF_3N_2$ $[M+H]^+$ m/z 363, found 363.1.

$^1$H NMR (400 MHz, DMSO-$d_6$) δ 9.82 (s, 1H), 8.20 (d, *J* =8.4Hz, 2H), 8.13 (d, *J* =8.8Hz, 1H), 7.85-7.66 (m, 2H), 7.43 (d, *J* =8.6Hz, 2H), 7.29 (t, J=7.8Hz, 1H), 7.15 (d, *J* =8.8Hz, 1H), 1.32-1.30 (m, 2H), 1.12-1.08 (m, 2H).

Example 5. 8-chloro-N-(4-(1-(trifluoromethyl)cyclopropyl)pyridin-2-yl)quinolin-2-amine (Compound 6)

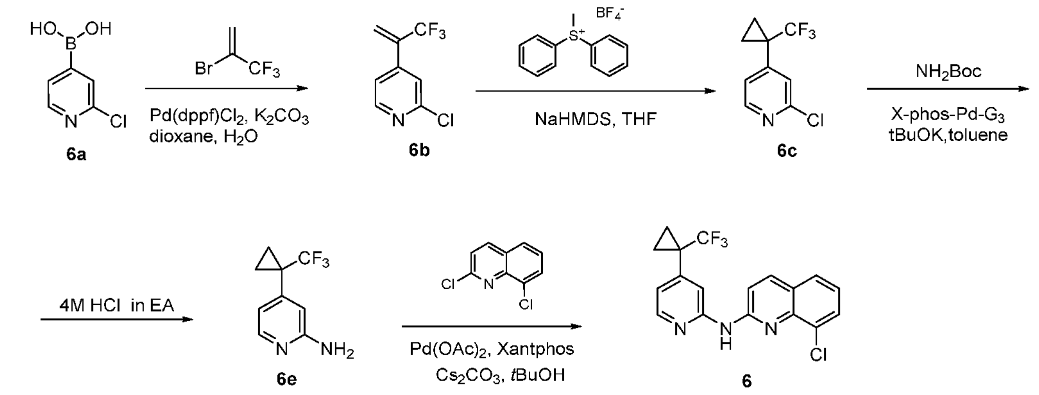


The preparation processes of Example 2 Steps 1-5 were followed to prepare the target compound of 8-chloro-N-(4-(1-(trifluoromethyl)cyclopropyl)pyridin-2-yl)quinolin-2-amine (Compound 6).
LCMS(ESI) calcd for $C_{18}H_{13}ClF_3N_3$ $[M+H]^+$ m/z 364.77, found 363.9.
$^1H$ NMR (400 MHz, DMSO-$d_6$) $\delta$ 10.44 (s,1H), 9.28 (s, 1H), 8.32 (d, *J =5.1* Hz, 1H), 8.22 (d, J=8.9 Hz, 1H), 7.78-7.84 (m, 2H), 7.43 (d, J=8.8 Hz, 1H), 7.31 (d, J=7.6 Hz, 1H), 7.12 (d, *J* =5.1 Hz, 1H), 1.43-1.46 (m, 2H), 1.26-1.29 (m, 2H).

Example 6. 8-chloro-N-(6-(1-(trifluoromethyl)cyclopropyl)pyridine-3-yl)quinolin-2-amine (Compound 7)

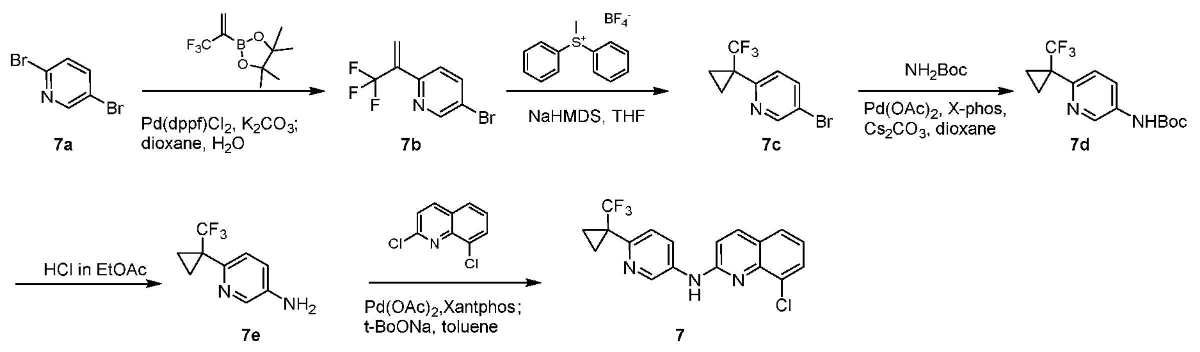


Step 1: 5-bromo-2-(3,3,3-trifluoroprop-1-en-2-yl)pyridine

At room temperature, under nitrogen protection, 2,5-dibromopyridine (7a) (5.00 g, 21.10 mmol), 4,4,5,5-tetramethyl-2-(3,3,3-trifluoroprop-1-en-2-yl)-1,3,2-dioxaborolane (4.41 g, 21.10 mmol), Pd(dppf)$Cl_2$ (1.54 g, 2.11 mmol) and potassium carbonate (8.75 g, 63.30 mmol) were added together to a mixed solution of dioxane and water. The reaction was carried out at 80°C for 12 hours until terminated. After cooling to room temperature, the mixture was filtered through celite and concentrated. The residue was extracted with ethyl acetate. The organic layer was washed with saturated brine, dried over anhydrous sodium sulfate, filtered and concentrated. The crude product was purified by silica gel column chromatography (eluent, PE:100%), to obtain the target 5-bromo-2-(3,3,3-trifluoroprop-1-en-2-yl)pyridine (7b).
LCMS(ESI) calcd for $C_8H_5BrF_3N$ $[M+H]^+$ m/z 252, found 252.9.
The remaining preparation processes were conducted by referring to the preparation process of Example 2 Steps 2-5, to obtain the target compound of 8-chloro-N-(6-(1-(trifluoromethyl)cyclopropyl)pyridine-3-yl)quinolin-2-amine (Compound 7).
LCMS(ESI) calcd for $C_{18}H_{13}ClF_3N_3$ $[M+H]^+$ m/z 364, found 364.0.
$^1H$ NMR (400 MHz, DMSO-$d_6$) $\delta$ 10.02 (s, 1H), 9.21 (d, *J =2.4* Hz, 1H), 8.75 (dd, *J* =8.8, 2.4 Hz, 1H), 8.20 (d, *J* =8.8 Hz, 1H), 7.85-7.71 (m, 2H), 7.54 (d, *J* =8.8 Hz, 1H), 7.33 *(t, J* =7.6 Hz, 1H), 7.18 (d, *J* =8.8 Hz, 1H), 1.39-1.32 (m, 4H).

Example 7. 8-chloro-N-(2-methoxy-4-(1-(trifluoromethyl)cyclopropyl)phenyl)quinolin-2-amine (Compound 8)

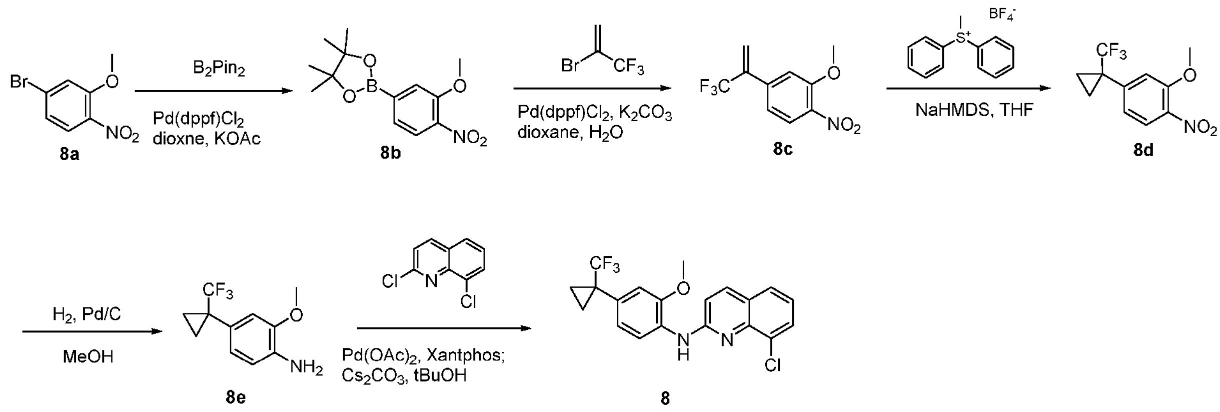


Step 1: 3-methoxy-4-nitrobenzeneboronic acid pinacol ester

At room temperature, 4-bromo-2-methoxy-1-nitrobenzene (8a) (3.00 g, 12.90 mmol), diboronic acid pinacol ester (4.91 g, 19.35 mmol), potassium acetate (2.53 g, 25.80 mmol) and $Pd(dppf)Cl_2$ (0.94 g, 1.29 mmol) were added to a dioxane solution (30 mL). The reaction was carried out by stirring at 90 °C under nitrogen atmosphere for 3 h until terminated. After cooling to room temperature, the mixture was filtered through celite. The filtrate was diluted with water and extracted with ethyl acetate, and the organic phase was dried and concentrated under reduced pressure. The crude product was purified by silica gel column chromatography (eluent, ethyl acetate: petroleum ether=1:10) to obtain the target compound of 3-methoxy-4-nitrobenzeneboronic acid pinacol ester(8b).
LCMS(ESI) calcd for $C_{13}H_{18}BNO_5$ $[M+H]^+$ m/z 280.1, found 280.0.

Step 2: 2-methoxy-1-nitro-4-(3,3,3-trifluoroallyl)benzene

At room temperature, under nitrogen atmosphere, 3-methoxy-4-nitrophenylboronic acid pinacol ester (8b) (2.80 g, 10.03 mmol), 2-bromo-3, 3, 3-trifluoropropene (7.04 g, 40.12 mmol), potassium carbonate (2.76 g, 20.06 mmol) and $Pd(dppf)Cl_2$ (0.73 g, 1.00 mmol) were added to dioxane (40 mL) and water (4 mL). The mixture was stirred at 70 °C to carry out the reaction for 3 hours until terminated. After cooling to room temperature, the mixture was filtered through celite. The filtrate was diluted with water and extracted with ethyl acetate, and the organic phase was dried and concentrated under reduced pressure. The crude product was purified by silica gel column chromatography (eluent, ethyl acetate: petroleum ether=1: 20) to obtain the target compound of 2-methoxy-1-nitro-4-(3,3,3-trifluoroallyl)benzene (8c).
$^1$H NMR (400 MHz, DMSO-$d_6$) δ 7.97 (d, *J* =8.4Hz, 1H), 7.40 (d, *J* =1.2 Hz, 1H), 7.20 (d, J=8.4 Hz, 1H), 6.40-6.34 (m, 1H), 6.27-6.32 (m, 1H), 3.99 (s, 3H).

Step 3: 2-methoxy-1-nitro-4-(1-(trifluoromethyl)cyclopropyl)benzene

At room temperature, 2-methoxy-1-nitro-4-(3,3,3-trifluoroallyl)benzene (8c) (0.50 g, 2.01 mmol) and methyl biphenylthiotetrafluoroborate (0.75 g, 2.62 mmol) were dissolved in 5 mL of tetrahydrofuran solution, and sodium bis(trimethylsilyl)amide (2 M in THF, 1.61 mL, 3.22 mmol) was added dropwise at 0°C. The mixture was stirred at room temperature to carry out the reaction for 3 hours until terminated. After the reaction was completed, the mixture was diluted with water and extracted with ethyl acetate, and the organic phase was dried and concentrated under reduced pressure. The crude product was purified by silica gel column chromatography (eluent, ethyl acetate: petroleum ether=1:20) to obtain the target compound of 2-methoxy-1-nitro-4-(1-(trifluoromethyl)cyclopropyl)benzene (8d).
$^1$H NMR (400 MHz, DMSO-$d_6$) δ 7.89 (d, *J* =8.4 Hz, 1H), 7.37 (d, *J =1.2Hz,* 1H), 7.22 (d, *J* =9.2 Hz, 1H), 3.96 (s, 3H), 1.37-1.43 (m, 2H), 1.25 -1.30 (m, 2H).

Step 4: 2-methoxy-4-(1-(trifluoromethyl)cyclopropyl)aniline

At room temperature, 2-methoxy-1-nitro-4-(1-(trifluoromethyl)cyclopropyl)benzene (8d) (0.42 g, 1.61 mmol) was dissolved in methanol solution (5 mL), 10% Pd/C (34.22 mg, 0.32 mmol) was added to the mixture, and hydrogen was replaced three times. The reaction was carried out by stirring at room temperature under a hydrogen atmosphere for 16

hours until terminated. The mixture was then filtered through celite and concentrated under vacuum to give the crude target compound of 2-methoxy-4-(1-(trifluoromethyl)cyclopropyl)aniline (8e).

LCMS(ESI) calcd for $C_{11}H_{12}F_3NO$ $[M+H]^+$ m/z 232.09, found 231.95.

Step 5: 8-chloro-N-(2-methoxy-4-(1-(trifluoromethyl)cyclopropyl)phenyl)quinolin-2-amine

At room temperature, 2-methoxy-4-(1-(trifluoromethyl)cyclopropyl)aniline (8e) (0.15 g, 0.65 mmol), 2,8-dichloroquinoline (0.13 g, 0.65 mmol), cesium carbonate (0.42 g, 1.30 mmol), Xantphos (0.04 g, 0.06 mmol) and palladium acetate (0.01 g, 0.06 mmol) were added to tert-butyl alcohol (5 mL). The mixture was stirred at 80 °C to carry out the reaction for 1 hour under a nitrogen atmosphere until terminated. After cooling to room temperature, the reaction solution was filtered through celite. The filtrate was diluted with water and extracted with ethyl acetate, dried over anhydrous sodium sulfate, and concentrated under reduced pressure. The crude product was purified by silica gel column chromatography (eluent, ethyl acetate: petroleum ether=1:20) to obtain the target compound of 8-chloro-N-(2-methoxy-4-(1-(trifluoromethyl)cyclopropyl)phenyl)quinolin-2-amine (Compound 8).

LCMS(ESI) calcd for $C_{20}H_{16}ClF_3N_2O$ $[M+H]^+$ m/z 393.09, found 392.95.

$^1$H NMR (400 MHz, DMSO-$d_6$) δ 9.23 (d, J=8.8 Hz, 1H), 8.87 (s, 1H), 8.12 (d, *J* =8. 8Hz, 1H), 7.77 (d, *J =7.2* Hz, 1H), 7.73 (d, *J* =8.0 Hz, 1H), 7.51 (d, *J* =8.8 Hz, 1H), 7.29 (t, *J =8.0* Hz, 1H), 7.12-7.05 (m, 2H), 3.94 (s, 3H), 1.28-1.35 (m, 2H), 1.14-1.19 (m, 2H).

Example 8. 8-chloro-N-(3-(1-(trifluoromethyl)cyclopropyl)phenyl)quinolin-2-amine (Compound 9)

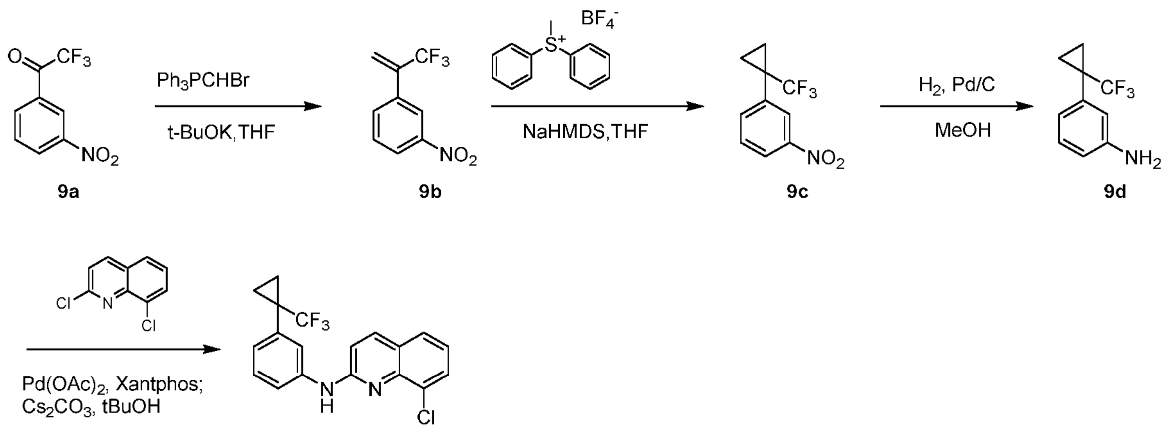


9a 9b 9c 9d

9

Step 1: 1-nitro-3-trifluoroallylbenzene

At 0°C, under nitrogen atmosphere, $Ph_3PCHBr$ (0.97 g, 2.72 mmol) was dissolved in THF (10 mL) and a solution of potassium tert-butoxide in tetrahydrofuran (1 M in THF, 2.72 mL, 2.72 mmol) was added dropwise. The mixture was stirred at 0°C for 1 hour. The reaction was carried out by adding 3'-nitro-2,2,2-trifluoroacetylbenzene (9a) (0.30 g, 1.36 mmol) and stirring at room temperature for 2 hours until terminated. The mixture was diluted with water and extracted with ethyl acetate, and the organic phase was dried and concentrated under reduced pressure. The crude product was purified by silica gel column chromatography (eluent, ethyl acetate: petroleum ether=1:35) to obtain the target compound of 1-nitro-3-trifluoroallylbenzene (9b).

$^1$H NMR (400 MHz, $CDCl_3$) δ 8.30-8.36 (m, 1H), 8.29-8.24 (m, 1H), 7.79 (d, *J* = 8.0Hz, 1H), 7.60 (t, *J* =8.0 Hz, 1H), 6.14 (d, *J* =0.8 Hz, 1H), 5.90-5.94 (m, 1H).

The remaining preparation processes were conducted by referring to the preparation processs of Example 7 Steps 3-5, to obtain the target compound of 8-chloro-N-(3-(1-(trifluoromethyl)propyl)phenyl)quinolin-2-amine (Compound 9).

LCMS(ESI) calcd for $C_{19}H_{14}ClF_3N_2$ $[M+H]^+$ m/z 363.08, found 363.00

$^1$H NMR (400 MHz, DMSO-$d_6$) δ 9.81 (s, 1H), 8.72 (s, 1H), 8.14 (d, *J* =8.8Hz, 1H), 7.86 (dd, *J* =8.0, 1.2Hz, 1H), 7.81-7.72 (m, 2H), 7.35 (t, *J* =8.0Hz, 1H), 7.29 (t, *J* =8.0Hz, 1H), 7.14 (d, *J* =8.8Hz, 1H), 7.08 (d, *J* =7.6Hz, 1H), 1.39-1.33 (m, 2H), 1.15-1.20 (m, 2H).

Example 9. 8-chloro-6-fluoro-N-(5-(1-(trifluoromethyl)cyclopropyl)pyridin-2-yl)quinolin-2-amine (Compound 10)

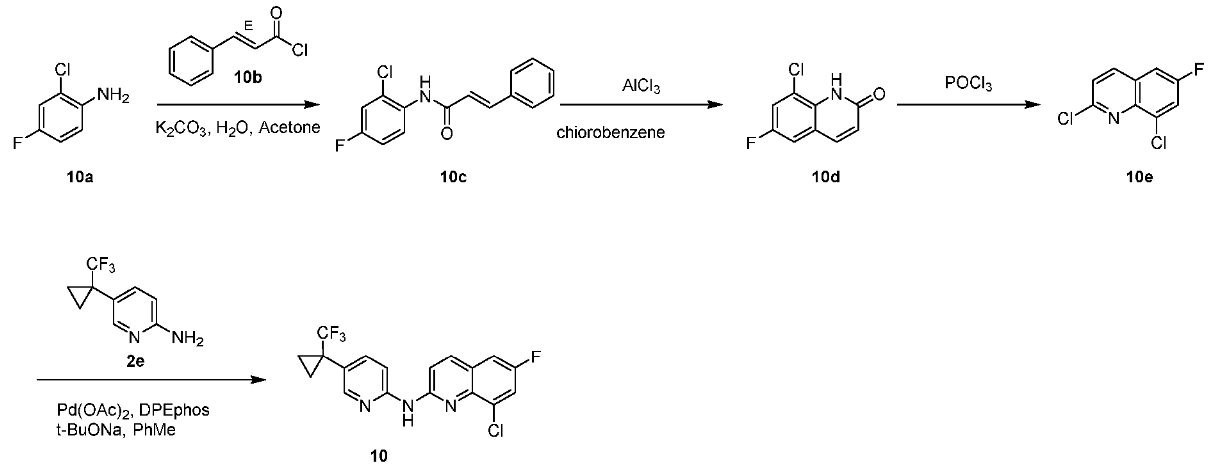


Step 1: (2E)-N-(2-chloro-4-fluorophenyl)-3-phenylprop-2-enamide

At room temperature, under nitrogen protection, 2-chloro-4-fluoroaniline (10a) (5.00 g, 0.03 mol) and $K_2CO_3$ (14.22 g, 0.10 mol) were added to water (26 mL). A solution of (2E)-3-phenylprop-2-enoyl chloride (10b) (5.71 g, 0.03 mol) in acetone (36 ml) was added dropwise at 0°C. The reaction was carried out at 25°C for 2 hours until terminated. The mixture was added with water and filtered to obtain the crude target compound of (2E)-N-(2-chloro-4-fluorophenyl)-3-phenylprop-2-enamide (10c).

$^1$H NMR (400 MHz, DMSO-$d_6$) δ 9.77 (s, 1H), 7.88 (dd, *J* = 9.0, 6.0 Hz, 1H), 7.68-7.57 (m, 3H), 7.54 (dd, *J* = 8.6, 2.8 Hz, 1H), 7.50-7.40 (m, 3H), 7.29-7.24(m, 1H), 7.06 (d, *J* = 15.8 Hz, 1H).

Step 2: 8-chloro-6-fluoroquinolin-2(1H)-one

At room temprature, under nitrogen protection, (2E)-N-(2-chloro-4-fluorophenyl)-3-phenylprop-2-enamide (10c) (3.00 g, 0.01 mol) and $AlCl_3$ (8.72 g, 0.07 mol) were added to chlorobenzene (10 ml). The reaction was carried out at 130°C for 4 hours until terminated. The mixture was added with water and filtered to obtain the crude target compound of 8-chloro-6-fluoroquinolin-2(1H)-one (10d).

$^1$H NMR (400 MHz, DMSO-$d_6$) δ 11.16 (s, 1H), 7.94 (d, *J* = 9.6 Hz, 1H), 7.74 (dd, *J* = 8.6, 2.8 Hz, 1H), 7.63 (dd, *J* = 8.8, 2.8 Hz, 1H), 6.68 (d, *J* = 9.6 Hz, 1H).

Step 3: 2,8-dichloro-6-fluoroquinoline

At room temprature, under nitrogen protection, 8-chloro-6-hydroxy-1H-quinolin-2-one(10d) (4.20 g, 0.02 mol) was added to $POCl_3$(30 mL). The reaction was carried out at 110°C for 4 hours until terminated. The mixture was added with water and filtered to obtain the crude target compound of 2,8-dichloro-6-fluoroquinoline (10e).

$^1$H NMR (400 MHz, DMSO-$d_6$) δ 8.53 (d, *J* = 8.6 Hz, 1H), 8.14-8.13 (m, 1H), 7.93 (dd, *J* = 8.8, 2.8 Hz, 1H), 7.77 (d, *J* = 8.6 Hz, 1H).

Step 4: 8-chloro-6-fluoro-N-(5-(1-(trifluoromethyl)cyclopropyl)pyridin-2-yl)quinolin-2-amine

At room temperature, under nitrogen protection, 5-(1-(trifluoromethyl)cyclopropyl)pyridin-2-amine (2e) (0.06 g, 0.30 mmol), 2,8-dichloro-6-fluoroquinoline (10e) (0.08 g, 0.36 mmol), palladium acetate (3.33 mg, 0.01 mmol), bis(2-diphenylphosphinophenyl) ether (8.00 mg, 0.01 mmol) and sodium tert-butoxide (0.09 g, 0.89 mmol) were added to ultra-dry toluene (30 mL). The reaction solution was stirred at 105°C to carry out the reaction for 10 hours under a nitrogen atmosphere until terminated. After the reaction solution cooled to room temperature, it was filtered through celite and the filtrate was concentrated in vacuo. The crude product was purified by silica gel column chromatography (eluent, petroleum ether: ethyl acetate=3: 1) to obtain the target compound of 8-chloro-6-fluoro-N-(5-(1-(trifluoromethyl)cyclopropyl)pyridin-2-yl)quinolin-2-amine (Compound 10).

LCMS(ESI) calcd for $C_{18}H_{12}ClF_4N_3$ $[M+H]^+$ m/z 382, found 382.0.

$^1$H NMR (400 MHz, DMSO-$d_6$) δ 10.49 (s, 1H), 8.96 (d, *J* =8.8Hz, 1H), 8.38 (d, *J* =2.4Hz, 1H), 8.21 (d, *J* =9.0Hz, 1H), 7.92-7.87 (m, 2H), 7.68 (dd, *J* =8.8, 2.8Hz, 1H), 7.54 (d, *J* =8.8Hz, 1H), 1.37-1.34 (m, 2H), 1.19-1.17 (m, 2H).

Example 10. 8-chloro-N-(2-(1-(trifluoromethyl)cyclopropyl)pyrimidin-5-yl)quinolin-2-amine (Compound 11)

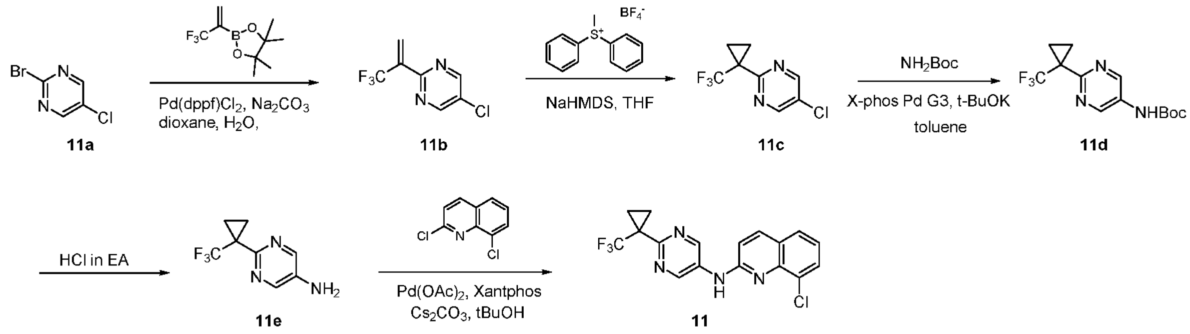


The preparation processes of Example 6 Steps 1-5 were followed to prepare the target compound of 8-chloro-N-(2-(1-(trifluoromethyl)cyclopropyl)pyrimidin-5-yl)quinolin-2-amine (Compound 11).

LCMS(ESI) calcd for $C_{17}H_{12}ClF_3N_4$ [M+H]$^+$ m/z 364.07, found 364.95.

$^1$H NMR (400 MHz, DMSO-$d_6$) δ 10.15 (s,1H), 9.54 (s, 2H), 8.25 (d, *J* =8.9 Hz, 1H), 7.88-7.77 (m, 2H), 7.36 (t, *J* =7.8 Hz, 1H), 7.21 (d,J =8.9 Hz, 1H), 1.52-1.48 (m, 4H).

Example 11. 8-chloro-N-(6-methyl-5-(1-(trifluoromethyl)cyclopropyl)pyridin-2-yl)quinolin-2-amine (Compound 12)

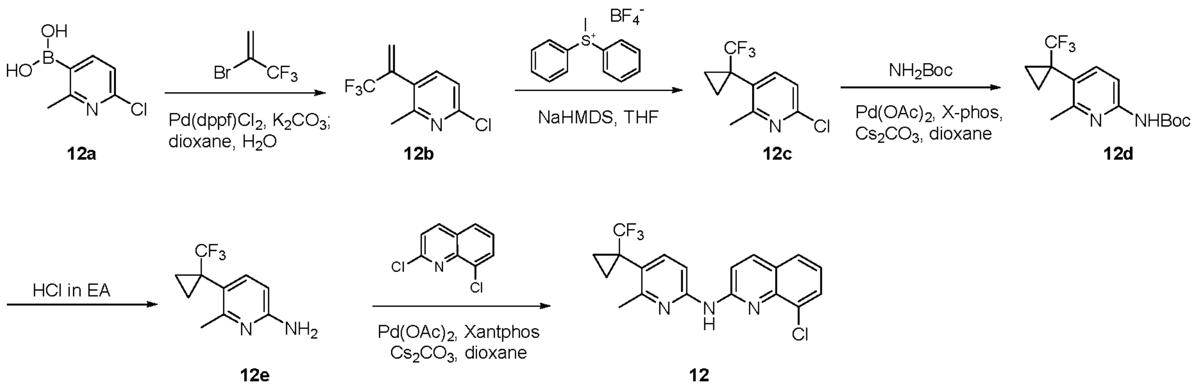


The preparation processes of Example 2 Steps 1-5 were followed to prepare the target compound of 8-chloro-N-(6-methyl-5-(1-(trifluoromethyl)cyclopropyl)pyridin-2-yl)quinolin-2-amine (Compound 12).

LCMS(ESI) calcd for $C_{19}H_{15}ClF_3N_3$ [M+H]$^+$ m/z 378.10, found 378.0.

$^1$H NMR (400 MHz, DMSO-$d_6$) δ 10.39 (s,1H), 8.90 (d, J=8.6Hz, 1H), 8.20 (d, *J* =9.0Hz, 1H), 7.77-7.83 (m, 3H), 7.47 (d, *J* =8.9Hz, 1H), 7.34 (t, *J* =7.8Hz, 1H), 2.55 (s, 3H), 1.43-1.46 (m, 2H), 1.18-1.23 (m, 2H).

Example 12. 8-chloro-N-(4-methyl-5-(1-(trifluoromethyl)cyclopropyl)pyridin-2-yl)quinolin-2-amine (Compound 13)

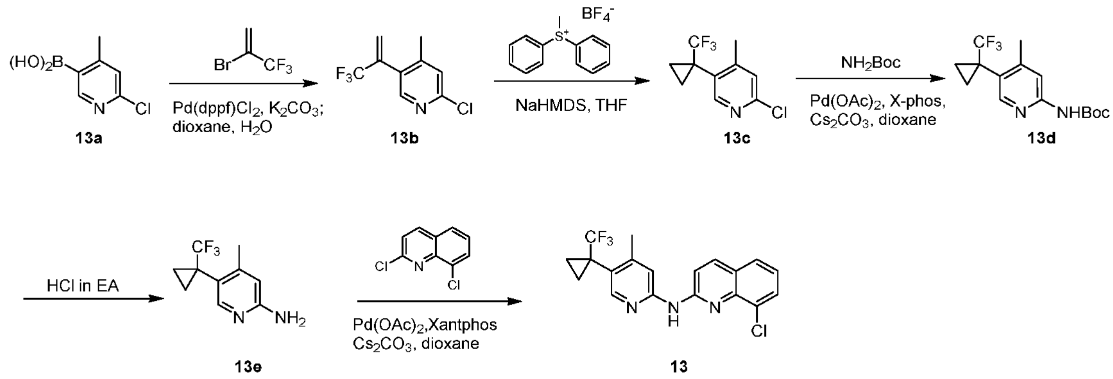


The preparation processes of Example 2 Steps 1-5 were followed to prepare the target Compound 8-chloro-N-(4-methyl-5-(1-(trifluoromethyl)cyclopropyl)pyridin-2-yl)quinolin-2-amine (Compound 13).

LCMS(ESI) calcd for $C_{19}H_{15}ClF_3N_3$ $[M+H]^+$ m/z 378, found 378.0.

$^1H$ NMR (400 MHz, DMSO-$d_6$) δ 9.03 (s,1H), 8.27 (s,1H), 8.22 (d, *J* =9.2Hz, 1H), 7.82-7.74 (m, 2H), 7.45 (t, *J* =7.6Hz, 1H), 7.35 (t, *J* =7.6Hz, 1H), 2.49 (s, 3H), 1.46-1.39 (m, 2H), 1.26-1.18 (m, 2H).

Example 13. 8-chloro-N-(5-(1-(trifluoromethyl)cyclopropyl)Pyrazin-2-yl)quinolin-2-amine (Compound 14)

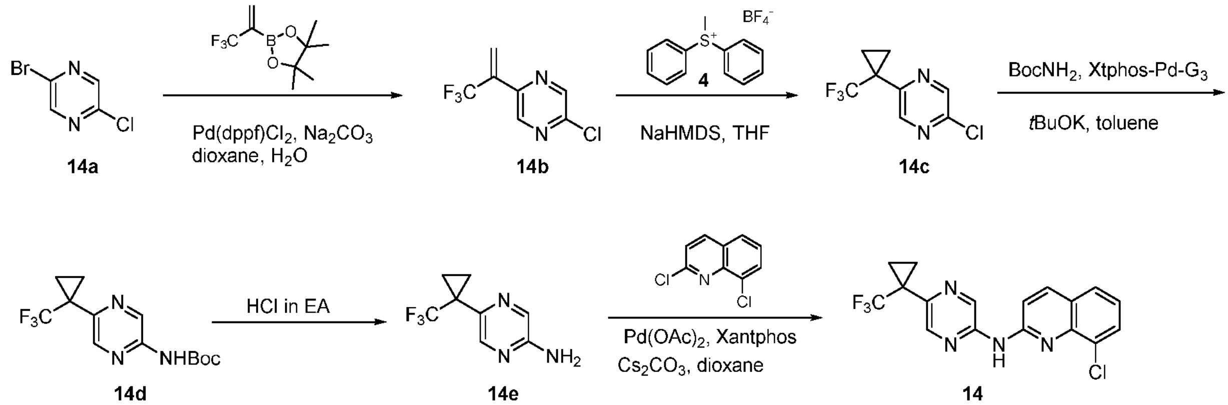


The preparation processes of Example 6 Steps 1-5 were followed to prepare the target compound of 8-chloro-N-(5-(1-(trifluoromethyl)cyclopropyl)Pyrazin-2-yl)quinolin-2-amine (Compound 14).

LCMS(ESI) calcd for $C_{17}H_{12}ClF_3N_4$ $[M+H]^+$ m/z 365.07, found 364.7.

$^1H$ NMR (400 MHz, DMSO-$d_6$) δ 10.75 (s, 1H), 10.26 (d, *J* =1.4Hz, 1H), 8.50 (s, 1H), 8.30 (d, *J* =8.9Hz, 1H), 7.82-7.88 (m, 2H), 7.50 (d, *J* =8.9Hz, 1H), 7.39 (t, *J* =7.8Hz, 1H), 1.42-1.45 (m, 2H), 1.36-1.39 (m, 2H).

Example 14. 8-chloro-N-(4-fluoro-5-(1-(trifluoromethyl)cyclopropyl)pyridin-2-yl)quinolin-2-amine (Compound 15)

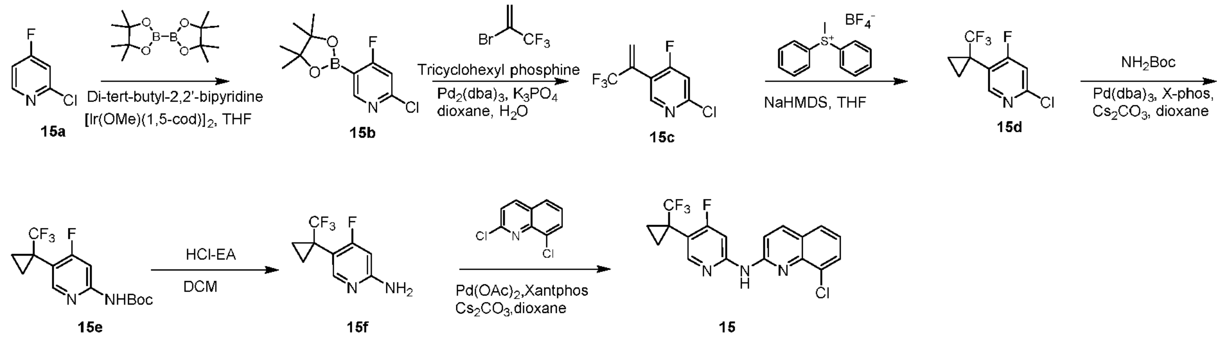

Step 1: 2-chloro-4-fluoro-5-(4, 4, 5, 5-tetramethyl-1,3,2-dioxaborane-2-yl)pyridine

At room temperature, under nitrogen atmosphere, 2-chloro-4-fluoropyridine (15a) (10.00 g, 76.00 mmol), biboronic acid pinacol ester (9.65 g, 38.00 mmol), 6,6'-di-tert-butyl-2,2'-bipyridine (0.20 g, 0.70 mmol), methoxy(cyclooctadiene)iridium dimer (0.25 g, 0.30 mmol) were added to tetrahydrofuran solution (120 mL). The reaction was carried out by stirring at 80°C for 16 hours until terminated. After concentration, the crude product was purified by silica gel column chromatography (eluent, ethyl acetate: petroleum ether=1:4), to obtain the target compound of 2-chloro-4-fluoro-5-(4, 4, 5, 5-tetramethyl-1, 3, 2-dioxaborane-2-yl)pyridine (15b).
LCMS(ESI) calcd for $C_{11}H_{14}BClFNO_2$ $[M+H]^+$ m/z 258.08, found 258.00
The remaining preparation processes were conducted by referring to the preparation processs of Example 2 Steps 1-5, to obtain the target compound of 8-chloro-N-(4-fluoro-5-(1-(trifluoromethyl)cyclopropyl)pyridin-2-yl)quinolin-2-amine (Compound 15).
LCMS(ESI) calcd for $C_{18}H_{12}ClF_4N_3$ $[M+H]^+$ m/z 382.07, found 381.90.
$^1H$ NMR (400 MHz, DMSO-$d_6$) δ 10.79 (s, 1H), 9.05 (d, *J* =14.0Hz, 1H), 8.42 (d, *J* =13.2Hz, 1H), 8.27 (d, *J* =9.2Hz, 1H), 7.78-7.92 (m, 2H), 7.45 (d, *J* =8.8Hz, 1H), 7.39 (t, *J* =8.0Hz, 1H), 1.38-1.48 (m, 2H), 1.21-1.26 (m, 2H).

Example 15. 8-chloro-2-((3-(1-(trifluoromethyl)cyclopropyl)phenyl)amino)quinolin-6-carbonitrile (Compound 16)

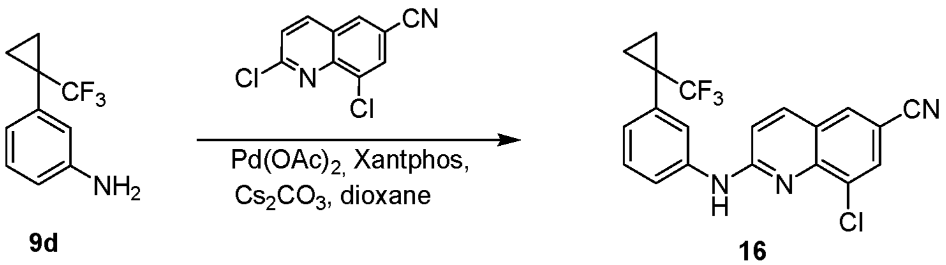


9d

16

At room temperature, 6-cyano-2, 8-dichloroquinoline (0.05 g, 0.22 mmol), 3-(1-(trifluoromethyl)cyclopropyl) aniline (9d) (0.06 g, 0.29 mmol), cesium carbonate (0.15 g, 0.45 mmol), Xantphos (0.01 g, 0.02 mmol), and palladium acetate (2.50 mg, 0.01 mmol) were dissolved in dioxane (5 mL) solution. The mixture was stirred at 80°C under nitrogen atmosphere to carry out the reaction for 2 h until terminated. The reaction solution was diluted with water and extracted with ethyl acetate, dried over anhydrous sodium sulfate, and concentrated under reduced pressure. The crude product was purified by silica gel column chromatography (eluent, ethyl acetate: petroleum ether=10: 1) to obtain the target compound of 8-chloro-2-((3-(1-(trifluoromethyl)cyclopropyl)phenyl)amino)quinolin-6-carbonitrile (Compound 16).
LCMS(ESI) calcd for $C_{20}H_{13}ClF_3N_3$ $[M+H]^+$ m/z 388.08, found 387.90.
$^1H$ NMR (400 MHz, DMSO-$d_6$) δ 10.20 (s, 1H), 8.71 (s, 1H), 8.35 (s, 1H), 8.19 (s, 2H), 7.85 (d, *J* =7.6Hz, 1H), 7.40-7.37 (m, 1H), 7.25 (d, J =8.8Hz, 1H), 7.15 (d, *J* =6.0Hz, 1H), 1.38-1.34 (m, 2H), 1.19-1.14 (m, 2H).

Example 16. 8-chloro-6-fluoro-N-(4-(1-(trifluoromethyl)cyclopropyl)pyridin-2-yl)quinolin-2-amine (Compound 17)

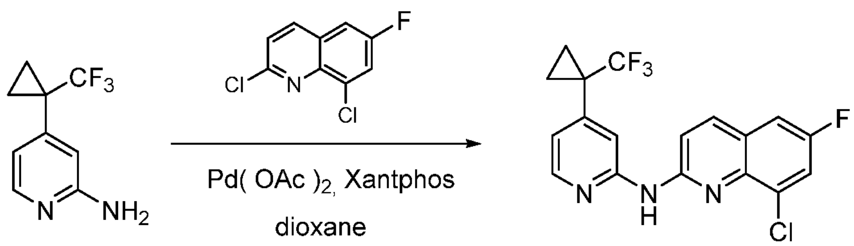


6e

17

The preparation process of Example 15 was followed to prepare the target compound of 8-chloro-6-fluoro-N-(4-(1-(trifluoromethyl)cyclopropyl)pyridin-2-yl)quinolin-2-amine (Compound 17).
LCMS(ESI) calcd for $C_{18}H_{12}ClF_4N_3$ $[M+H]^+$ m/z 382.07, found 381.9.
$^1H$ NMR (400 MHz, DMSO-$d_6$) δ 10.46 (s, 1H), 9.23 (s, 1H), 8.32 (d, *J* =5.1Hz, 1H), 8.21 (d, *J* =9.0Hz, 1H), 7.89

(dd, $J$ =8.7, 2.7Hz, 1H), 7.68 (dd, $J$ =8.9, 2.7Hz, 1H), 7.48 (d, $J$ =8.9Hz, 1H), 7.12 (d, $J$ =4.9Hz, 1H), 1.46-1.43 (m, 2H), 1.29-1.26 (m, 2H).

Example 17. 8-chloro-6-fluoro-N-(4-(1-(trifluoromethyl)cyclopropyl)phenyl)quinolin-2-amine (Compound 18)

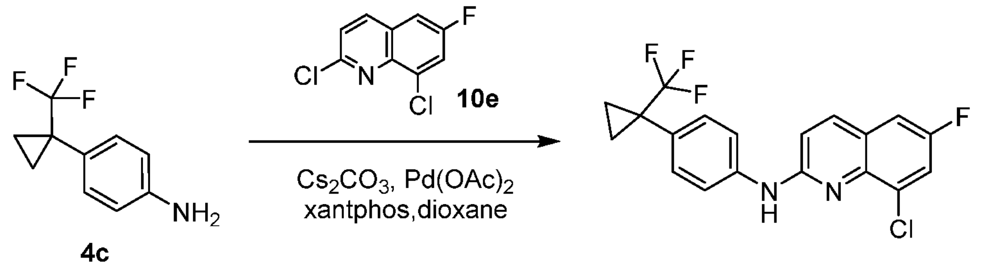


4c

18

The preparation process of Example 15 was followed to prepare the target compound of 8-chloro-6-fluoro-N-(4-(1-(trifluoromethyl)cyclopropyl)phenyl)quinolin-2-amine (Compound 18).
LCMS(ESI) calcd for $C_{19}H_{13}ClF_4N_2$ $[M+H]^+$ m/z 381.07, found 380.90.
$^1$H NMR (400 MHz, DMSO-$d_6$) δ 9.83 (s, 1H), 8.17 (d, $J$ =8.8Hz, 2H), 8.13 (d, $J$ =9.2Hz, 1H), 7.83 (dd, $J$ =8.4, 2.8Hz, 1H), 7.63 (dd, $J$ =9.2, 2.8Hz, 1H), 7.43 (d, $J$ =8.4Hz, 2H), 7.20 (d, $J$ =8.8Hz, 1H), 1.28-1.33 (m, 2H), 1.08-1.14 (m, 2H).

Example 18. 8-chloro-2-((5-(1-(trifluoromethyl)cyclopropyl)pyridin-2-yl)amino)quinolin-6-nitrile (Compound 19)

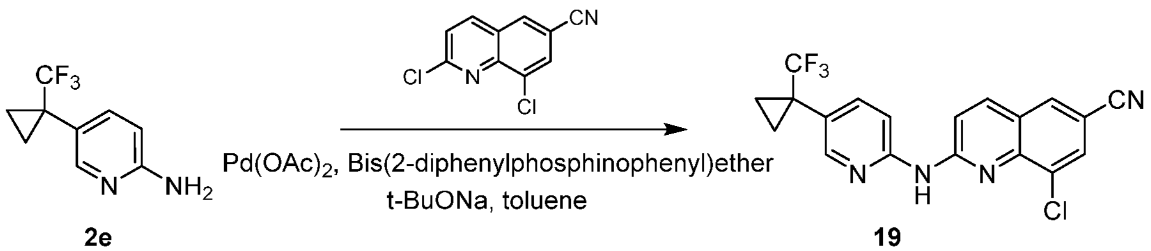


2e

19

At room temperature, under nitrogen protection, 5-(1-(trifluoromethyl)cyclopropyl)pyridin-2-amine (2e) (0.04 g, 0.20 mmol), 2,8-dichloroquinolin-6-carbonitrile (0.02 g, 0.10 mmol), palladium acetate (2.22 mg, 0.01 mmol), bis(2-diphenylphosphinophenyl) ether (5.33 mg, 0.01 mmol) and sodium tert-butoxide (0.06 g, 0.59 mmol) were added to ultra-dry toluene (40 mL). The reaction solution was stirred at 90 °C to carry out the reaction for 2 h under a nitrogen atmosphere until terminated. After the reaction solution cooled to room temperature, it was filtered through celite and the filtrate was concentrated in vacuo. The crude product was purified by reverse phase prep-HPLC (eluent, acetonitrile: water(0.05% ammonia)) to obtain the target compound of 8-chloro-2-((5-(1-(trifluoromethyl)cyclopropyl)pyridin-2-yl)amino)quinolin-6-nitrile(Compound 19).
LCMS(ESI) calcd for $C_{19}H_{12}ClF_3N_4$ $[M+H]^+$ m/z 389, found 388.9.
$^1$H NMR (400 MHz, DMSO-$d_6$) δ 10.80 (s, 1H), 8.97 (d, $J$ =8.8Hz, 1H), 8.41 (dd, $J$ =12.0, 1.6Hz, 2H), 8.29 (d, $J$ =9.0Hz, 1H), 8.24 (d, $J$ =1.6Hz, 1H), 7.96 (dd, $J$ =8.6, 2.2Hz, 1H), 7.60 (d, $J$ =9.0Hz, 1H), 1.39-1.36 (m, 2H), 1.24-1.21 (m, 2H).

Example 19. 8-chloro-2-((4-(1-(trifluoromethyl)cyclopropyl)phenyl)amino)quinolin-6-carbonitrile (Compound 20)

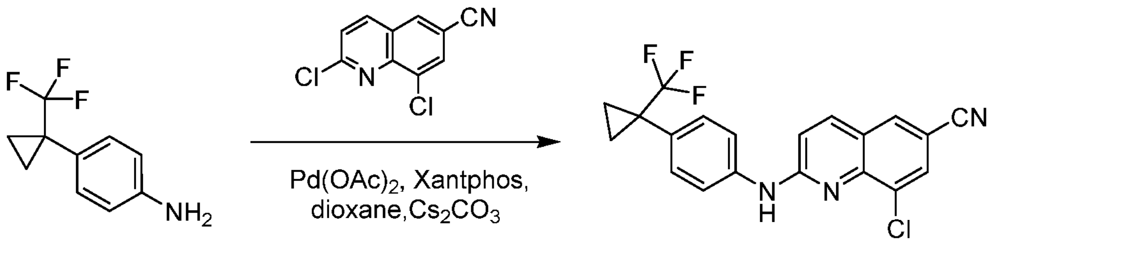


4c 20

The preparation process of Example 15 was followed to prepare the target compound of 8-chloro-2-((4-(1-(trifluoromethyl)cyclopropyl)phenyl)amino)quinolin-6-carbonitrile (Compound 20).

LCMS(ESI) calcd for $C_{20}H_{13}ClF_3N_3$ $[M+H]^+$ m/z 388.08, found 388.00.

$^1$H NMR (400 MHz, DMSO-$d_6$) δ10.20 (s, 1H), 8.35 (d, *J* =2.0Hz, 1H), 8.24-8.15 (m, 4H), 7.47 (d, *J* =8.4Hz, 2H), 7.26 (d, *J* =8.8Hz, 1H), 1.33-1.30 (m, 2H), 1.13-1.10 (m, 2H).

Example 20. 8-chloro-6-(methylsulfonyl)-N-(4-(1-(trifluoromethyl)cyclopropyl)phenylquinolin-2-amine (Compound 21)

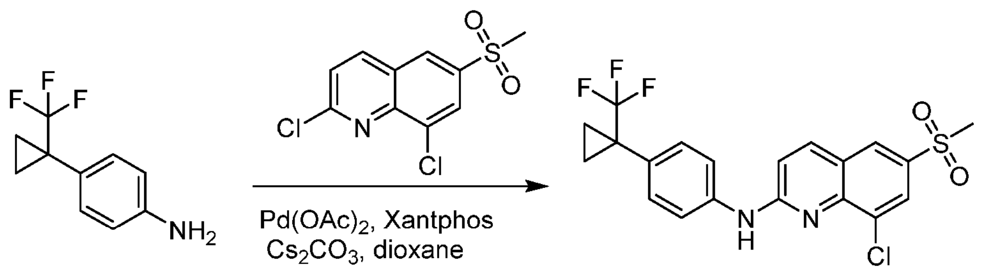


4c 21

The preparation process of Example 15 was followed to prepare the target compound of 8-chloro-6-(methylsulfonyl)-N-(4-(1-(trifluoromethyl)cyclopropyl)phenylquinolin-2-amine (Compound 21).

LCMS(ESI) calcd for $C_{20}H_{16}ClF_3N_2O_2S$ $[M+H]^+$ m/z 441.06, found 441.00.

$^1$H NMR (400 MHz, $CDCl_3$) δ 8.20-8.19 (m, 2H), 8.04 (d, *J* =9.2Hz, 1H), 7.80 (s, 2H), 7.52 (d, *J* =8.4Hz, 2H), 7.08 (d, *J* =8.4Hz, 1H), 3.14 (s,3H), 1.37-1.32 (m,2H), 1.06-1.02 (m,2H).

Example 21. 8-chloro-N-(4-(1,1-difluoro-2-methylpropan-2-yl)phenyl)quinolin-2-amine (Compound 22)

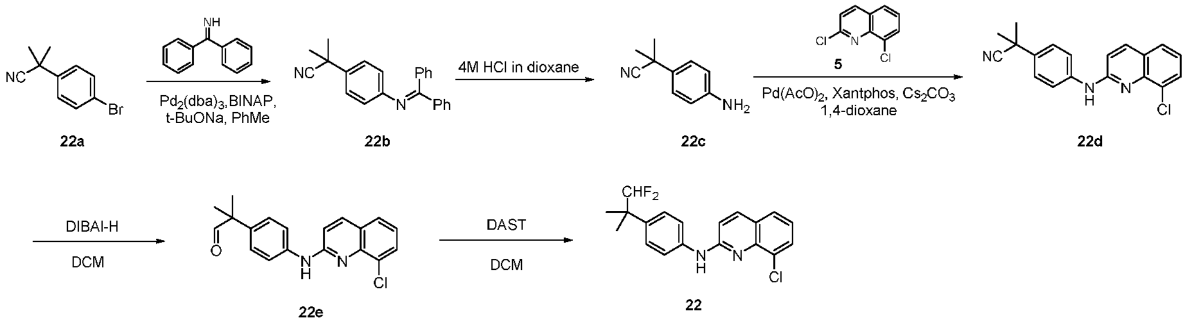


22a 22b 22c 22d 22e 22

Step 1: 2-(4-((diphenylmethylene)amino)phenyl)-2-methylpropionitrile

At room temperature, under nitrogen protection, 2-(4-bromophenyl)-2-methylpropionitrile (22a) (0.50 g, 2.23 mmol), benzophenone imine (0.49 g, 2.68 mmol), $Pd_2(dba)_3$ (0.04 g, 0.04 mmol), BINAP (0.08 g, 0.13 mmol) and sodium

tert-butoxide (0.32 g, 3.35 mmol) were added to a toluene solution (8 mL). The mixture was stirred at 90 °C for 17 hours under nitrogen atmosphere. After cooling to room temperature, the reaction solution was filtered through celite and the filtrate was concentrated in vacuo. The crude product was purified by silica gel column chromatography (eluent, petroleum ether: ethyl acetate=9: 1) to obtain the target compound of 2-(4-((diphenylmethylene)amino)phenyl)-2-methylpropionitrile (22b).

LCMS(ESI) calcd for $C_{23}H_{20}N_2$ $[M+H]^+$ m/z 325, found 325.2.

Step 2: 2-(4-aminophenyl)-2-methylpropionitrile

At room temperature, 2-(4-((diphenylmethylene)amino)phenyl)-2-methylpropionitrile(22b)(0.70 g, 2.16 mmol) was dissolved in ethyl acetate (3.5 mL), and ethyl acetate solution (4 M, 7 mL) was added. The solution was reacted at room temperature for 15 minutes until terminated. The mixture was extracted with ethyl acetate and the pH of the aqueous phase was adjusted to 9 by saturated sodium bicarbonate solution. The resultant was extracted with dichloromethane, dried, filtered and concentrated to obtain the crude target compound 2-(4-aminophenyl)-2-methylpropionitrile (22c).

LCMS(ESI) calcd for $C_{10}H_{12}N_2$ $[M+H]^+$ m/z 161, found 161.0.

Step 3: 2-(4-((8-chloroquinolin-2-yl)amino)phenyl)-2-methylpropionitrile

At room temperature, under the protection of nitrogen, 2-(4-aminophenyl)-2-methylpropionitrile (22c) (0.20 g, 1.25 mmol), 2, 8-dichloroquinoline (0.30 g, 1.50 mmol), palladium acetate (0.01 g, 0.06 mmol), Xantphos (0.03 g, 0.06 mmol) and cesium carbonate (1.22 g, 3.74 mmol) were added to a 1,4-dioxane solution (120 mL). The reaction was carried out by stirring at 90 °C for 3 h under nitrogen atmosphere until terminated. After cooling to room temperature, the reaction solution was filtered through celite and the filtrate was concentrated in vacuo. The crude product was purified by silica gel column chromatography (eluent, petroleum ether: ethyl acetate=3: 1) to obtain the target compound of 2-(4-((8-chloroquinolin-2-yl)amino)phenyl)-2-methylpropionitrile (22d).

LCMS(ESI) calcd for $C_{19}H_{16}ClN_3$ $[M+H]^+$ m/z 322, found 322.1.

Step 4: 2-(4-((8-chloroquinolin-2-yl)amino)phenyl)-2-methylpropane

At -78°C, under nitrogen protection, 2-(4-((8-chloroquinolin-2-yl)amino)phenyl)-2-methylpropionitrile (22d) (0.10 g, 0.31 mmol) was dissolved in ultra-dry dichloromethane solution (2.5 mL), and DIBAL-H (1 M, 0.4 mL, 0.37 mmol) was added dropwise. After the addition was complete, the mixture was stirred at -78 °C for 2 hours and then warmed to room temperature and stirred for 2 hours to terminate the reaction. Under ice bath, the reaction was quenched with 1 M hydrochloric acid solution (1.2 mL). The resultant was extracted by addition of dichloromethane, washed, dried, filtered and concentrated. The crude product was purified by silica gel column chromatography (eluent, petroleum ether: ethyl acetate=3: 1) to obtain the target compound of 2-(4-((8-chloroquinolin-2-yl)amino)phenyl)-2-methylpropane(22e).

LCMS(ESI) calcd for $C_{19}H_{17}ClN_2O$ $[M+H]^+$ m/z 325, found 324.9.

Step 5: 8-chloro-N-(4-(1,1-difluoro-2-methylpropan-2-yl)phenyl)quinolin-2-amine

At room temperature, 2-(4-((8-chloroquinolin-2-yl)amino)phenyl)-2-methylpropane (22e) (0.08 g, 0.26 mmol) was dissolved in dichloromethane (8 mL). The temperature was lowered to -20°C, and DAST (0.11 g, 0.65 mmol) was added dropwise. The reaction solution was stirred at -20 °C for 10 minutes and then warmed to room temperature for 2 hours to terminate the reaction. The reaction was quenched by adding water. The mixture was adjusted to pH 8-9 with saturated sodium bicarbonate solution. The resultant was extracted with ethyl acetate, washed, dried, filtered, and concentrated. The crude product was purified by reverse phase prep-HPLC (eluent, acetonitrile/water(0.05%ammonia)) to obtain the target compound of 8-chloro-N-(4-(1,1-difluoro-2-methylpropan-2-yl)phenyl)quinolin-2-amine (Compound 22).

LCMS(ESI) calcd for $C_{19}H_{17}ClF_2N_2$ $[M+H]^+$ m/z 347, found 346.9.

$^1$H NMR (400 MHz, DMSO-$d_6$) $\delta$ 9.84 (s,1H), 8.23 (d,J =8.4Hz,2H), 8.15 (d, *J* =9.2Hz, 1H), 7.89-7.65 (m, 2H), 7.39 (d, *J*=8.4Hz, 2H), 7.29 (t, *J*=7.8Hz, 1H), 7.17 (d,J =8.8Hz, 1H), 5.48 (dd, *J*=44.8, 17.6Hz, 1H), 1.38 (d,J =21.6Hz,3H), 1.28 (d, *J* =22.0Hz, 3H).

Example 22. 8-chloro-N-(6-(1-(trifluoromethyl)cyclopropyl)pyridazin-3-yl)quinolin-2-amine (Compound 23)

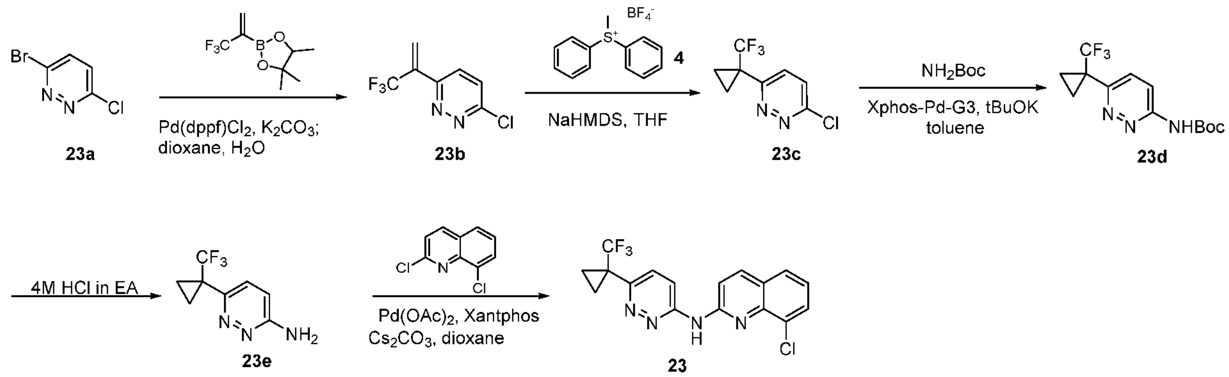


The preparation processes of Example 6 Steps 1-5 were followed to prepare the target compound of 8-chloro-N-(6-(1-(trifluoromethyl)cyclopropyl)pyridazin-3-yl)quinolin-2-amine (Compound 23).

LCMS(ESI) calcd for $C_{17}H_{12}ClF_3N_4$ $[M+H]^+$ m/z 365.07, found 365.0.

$^1H$ NMR (400 MHz, DMSO-$d_6$) δ 11.03 (s,1H), 9.30 (d, *J* =9.4Hz, 1H), 8.30 (d, *J* =8.9Hz, 1H), 7.82-7.90 (m, 3H), 7.54 (d, *J* =8.9Hz, 1H), 7.39 (t, *J* =7.8Hz, 1H), 1.42-1.50 (m, 4H).

Example 23. 8-chloro-N-(5-(1-(trifluoromethyl)cyclopropyl)pyrimidin-2-yl)quinolin-2-amine (Compound 24)

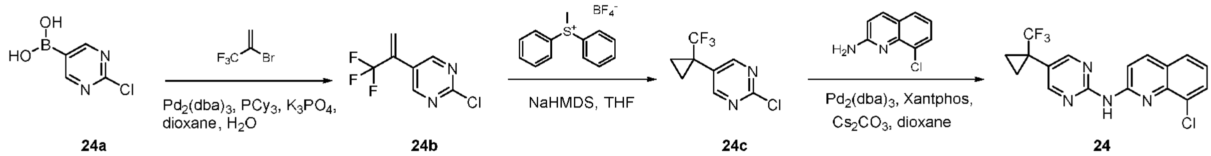


Step 1: 2-chloro-5-(3,3,3-trifluoroprop-1-en-2-yl)pyrimidine

At room temperature, under nitrogen protection, (2-chloropyrimidin-5-yl)boronic acid (24a) (8.00 g, 50.63 mmol), 2-bromo-3,3,3,3-trifluoroprop-1-ene (13.29 g, 75.95 mmol), $Pd_2(dba)_3$ (2.32 g, 2.53 mmol), tricyclohexylphosphine (1.42 g, 5.06 mmol) and potassium phosphate (21.47 g, 101.26 mmol) were added together to a mixed solution of dioxane and water. The reaction was carried out at 80°C for 2 hours until terminated. After cooling to room temperature, the mixture was filtered through celite and concentrated. The residue was extracted with ethyl acetate. The organic layer was washed with saturated brine, dried over anhydrous sodium sulfate, filtered and concentrated. The crude product was purified by silica gel column chromatography (eluent, petroleum ether: ethyl acetate=10:1), to obtain the target compound of 2-chloro-5-(3,3,3-trifluoroprop-1-en-2-yl)pyrimidine (24b).

$^1H$ NMR (400 MHz, $CDCl_3$) δ 8.71 (s, 2H), 6.25-6.16 (m, 1H), 5.99-5.92 (m, 1H).

Step 2: 2-chloro-5-(1-(trifluoromethyl)cyclopropyl)pyrimidine

At 0°C, under nitrogen protection, NaHMDS (2 M in THF, 8.5 mL, 16.93 mmol) was added dropwise to a solution of 2-chloro-5-(3,3,3-trifluoroprop-1-en-2-yl)pyrimidine (24b) (2.20 g, 10.58 mmol) and diphenyl(methyl)sulfonium tetrafluoroborate (3.96 g, 13.75 mmol) in anhydrous tetrahydrofuran. The mixture was stirred at room temperature to carry out the reaction for 3 hours until terminated. After the reaction was completed, the mixture was quenched with ice water and extracted with ethyl acetate. The organic layer was washed with saturated brine, dried over anhydrous sodium sulfate, filtered and concentrated. The crude product was purified by silica gel column chromatography (eluent, petroleum ether:ethyl acetate=10:1), to obtain the target compound of 2-chloro-5-(1-(trifluoromethyl)cyclopropyl)pyrimidine (24c).

$^1H$ NMR (400 MHz, DMSO-$d_6$) δ 8.75 (d, *J* =1.3Hz,2H), 1.30-1.24 (m, 2H), 1.16 (t, *J* =6.1Hz, 2H) .

Step 3: 8-chloro-N-(5-(1-(trifluoromethyl)cyclopropyl)pyrimidin-2-yl)quinolin-2-amine

At room temperature, under nitrogen protection, 8-chloroquinolin-2-amne (0.05 g, 0.28 mmol), 2-chloro-5-(1-(trifluoromethyl)cyclopropyl)pyrimidine (24c) (0.05 g, 0.23 mmol), $Pd_2(dba)_3$ (0.03 g, 0.03 mmol), 4,5-bis(diphenylphosphino)-9,9-dimethylxanthene (0.02 g, 0.03 mmol) and cesium carbonate (0.18 g, 0.56 mmol) were dissolved in dioxane solution. The reaction was carried out by stirring at 110 °C for 4 hours until terminated. After cooling to room temperature,

the mixture was filtered through celite and concentrated. The residue was extracted with ethyl acetate, and the organic layer was washed with saturated brine, dried over anhydrous sodium sulfate, filtered and concentrated. The crude product was purified by high performance liquid chromatography (ZORBAX ECLIPES PLUS C18, 1.8um, 4.6*50 mm, eluting with 5% to 95% MeCN/$H_2O$ containing 0.1% FA), to obtain the target compound of 8-chloro-N-(5-(1-(trifluoromethyl)cyclopropyl)pyrimidin-2-yl)quinolin-2-amine (Compound 24).

LCMS(ESI) calcd for $C_{17}H_{13}ClF_3N_4$ $[M+H]^+$ m/z 365.08, found 364.95.

$^1H$ NMR (400 MHz, DMSO-$d_6$) δ 10.44 (s, 1H), 8.68 (s, 2H), 8.50 (d, *J* =9.0Hz, 1H), 8.39 (d, *J* =9.1Hz, 1H), 7.86 (dd, *J* =12.2, 4.6Hz, 2H), 7.41 (t, *J* =7.8Hz, 1H), 1.39-1.37 (m, 2H), 1.26-1.21 (m, 2H).

Example 24. (2S, 3S, 4S, 5R, 6R)-6-((8-chloroquinolin-2-yl)(4-(1-(trifluoromethyl)cyclopropyl)phenyl)amino)-3,4,5-trihydroxytetrahydro-2H-pyran-2-carboxylic acid (Compound 25)

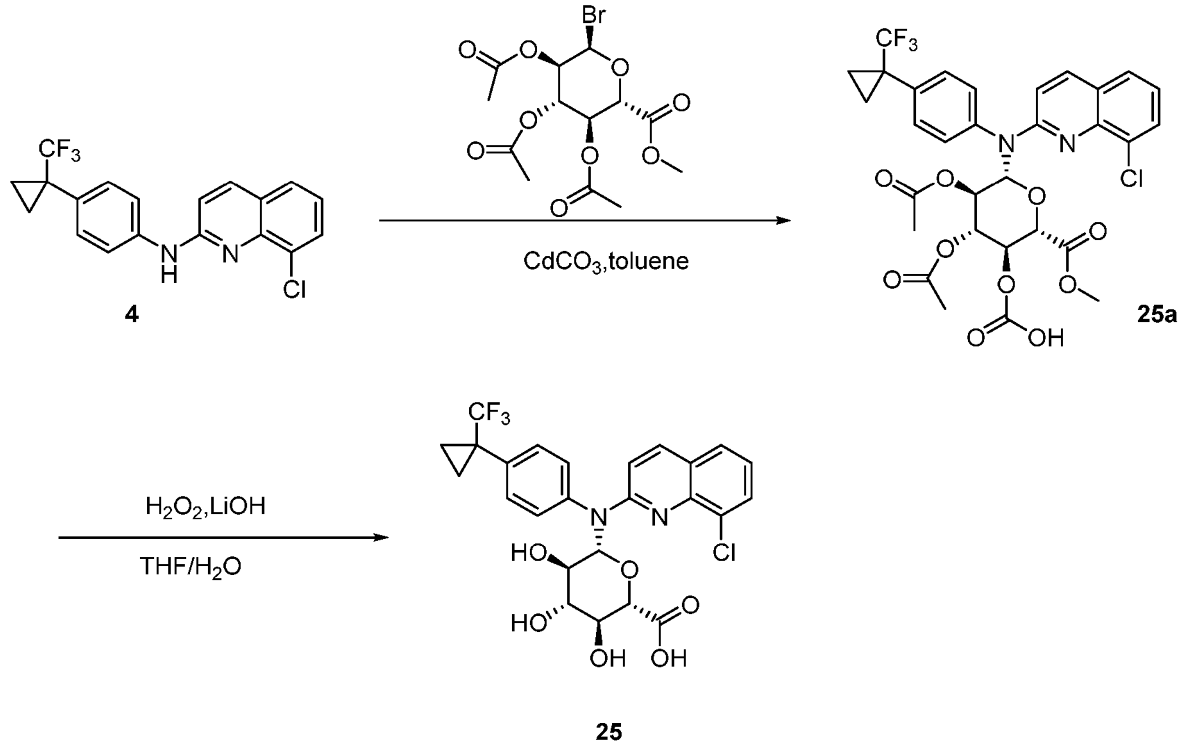


Step 1: (2R,3R,4S,5S,6S)-5-(carboxyoxo)-2-((8-chloroquinolin-2-yl)(4-(1-(trifluoromethyl)cyclopropyl)phenyl)amino)-6-(methoxycarbonyl)tetrahydro-2H-pyran-3,4-diyl diacetate

At room temperature, under nitrogen protection, 8-chloro-N-(4-(1-(trifluoromethyl)cyclopropyl)phenyl)quinolin-2-amine (Compound 4) (0.50 g, 1.38 mmol) and cadmium carbonate (0.15 g, 0.90 mmol) were added to toluene (20 mL). After reflux at 140°C for 12 hours, (2R,3R,4S,5S,6S)-2-bromo-6-(methoxycarbonyl)tetrahydro-2H-pyran-3,4,5-triol triacetate (1.05 g, 2.64 mmol) was added. The reaction was terminated after 24 hours at 140°C. After cooling to room temperature, the mixture was concentrated under reduced pressure. The crude product was purified by silica gel column chromatography (eluent, dichloromethane:methanol=20: 1) to obtain target compound of (2R,3R,4S,5S,6S)-5-(carboxyoxo)-2-((8-chloroquinolin-2-yl)(4-(1-(trifluoromethyl)cyclopropyl)phenyl)amino)-6-(methoxycarbonyl)tetrahydro-2H-pyran-3,4-diyl diacetate (25a).

LCMS(ESI) calcd for $C_{31}H_{28}ClF_3N_2O_{10}$ $[M+H]^+$ m/z 681.14, found 681.10.

Step 2: (2S,3S,4S,5R,6R)-6-((8-chloroquinolin-2-yl)(4-(1-(trifluoromethyl)cyclopropyl)phenyl)amino)-3,4,5-trihydroxytetrahydro-2H-pyran-2-carboxylic acid

At room temperature, Lithium hydroxide monohydrate (0.45 g, 10.74 mmol) was dissolved in water (15 mL), hydrogen peroxide (1 mL) was added, and stirred at room temperature for 10 minutes. (2R,3R,4S,5S,6S)-5-(carboxyoxo)-2-((8-chloroquinolin-2-yl)(4-(1-(trifluoromethyl)cyclopropyl)phenyl)amino)-6-(methoxycarbonyl)tetrahydro-2H-pyran-3,4-diyl diacetate (25a) (0.30 g, 0.44 mmol) was dissolved in tetrahydrofuran (15 mL). The prepared lithium hydroxide/hydrogen peroxide mixed solution was slowly added dropwise at room temperature and stirred at room temperature to carry out the reaction for 2 hours until terminated. The mixture was quenched with saturated sodium thiosulfate, with pH adjusted to 3 with dilute hydrochloric acid, and extracted with ethyl acetate. The organic phase was washed with water, filtered, dried and concentrated. The crude product was purified by high performance liquid chromatography (ZORBAX ECLIPES PLUS C18, 1.8um, 4.6*50 mm, eluting with 5% to 95% MeCN/$H_2O$ containing 0.1% FA), to obtain the target compound of (2S,3S,4S,5R,6R)-6-((8-chloroquinolin-2-yl)(4-(1-(trifluoromethyl)cyclopropyl)phenyl)amino)-3,4,5-trihydroxytetrahydro-2H-pyran-2-carboxylic acid (Compound 25).

LCMS (ESI) calcd for $C_{31}H_{28}ClF_3N_2O_{10}$ $[M + H]^+$ m/z 539.11, found 539.04.

Pharmacological activity test: Regulation of miR-124 expression by the quinoline derivatives in a PBMC in vitro model

A Materials and Procedures

1. Materials

a. human PBMC cells
b. PHA-L
c. IL-2
d. mirVana™ miRNA Extraction kit
e. TaqMan Advanced miRNA cDNA Synthesis Kit
f. Taqman Fast Gene Expression Master Mix
g. TaqMan Advanced miRNA Assay (mir191)
h. TaqMan Advanced miRNA Assay (mir124)

2. Procedures

Human PBMC cells were resuscitated. The cell suspension was transferred to a 75 $cm^2$ culture flask containing 13 mL of culture medium. The cells were cultured overnight in a 37°C, 5% $CO_2$ incubator. The cell suspension was then collected in a conical flask, centrifuged at 1200 rpm for 5 minutes, and the supernatant was discarded. The cells were resuspended in 1 mL of fresh culture medium, and then the cell clumps were gently blown apart and inoculated into a 6-well plate at a density of $4*10^6$ cells/mL. 5 μg/mL PHA-L and 10 ng/mL IL-2 were added to each well. The cells were incubated in the incubator for 48 hours. 5 μM of the test compound was added to each well of a 6-well plate, and 0.1% DMSO was added to the blank control wells, and the plate was incubated at 37°C for 24 h. Cells were collected, Total RNA was isolated and extracted according to the procedure of the mirVana™ miRNA Extraction Kit, and cDNA was prepared according to the procedure of the TaqMan Advanced miRNA cDNA Synthesis Kit. MiR-191 was used as the internal reference gene for qPCR test. The qPCR reaction system is shown in Table 1.
That is, RNA sample → poly (A) tailing reaction → adapter reaction → reverse transcription (RT) reaction → miR-amplification reaction → qPCR test.

Table 1 qPAR reaction system

| | |
|---|---|
| 2×Taqman Fast Gene Expression Master Mix | 5 μL |
| 20×Primer/Probe for miR-124 or miR-191 | 0.5 μL |
| cDNA template and $H_2O$ | 4.5 μL |
| Total volume | 10 μL |

The qPCR procedure includes: 50°C 2min; 95°C 20s; 95° C 1s , 60°C 20s, 40 cycles.

3. Data analysis

The relative gene expression was calculated using the following formula: $\Delta Ct$ = Ct (miR-124) -Ct (miR-191)

Relative miRNA expression= $2^{-\Delta Ct}$.

4, Results

The relative expression levels of miR-124 detected for each test compound are shown in Table 2.

Table 2 Relative expression of miR-124 of each test compound

| Compound NO | Fold change compared with DMSO-treated cells |
|---|---|
| DMSO | 1.00 |
| 1 | 1.50 |
| 2 | 8.35 |
| 3 | 7.33 |
| 4 | 15.93 |
| 6 | 7.28 |
| 7 | 1.80 |
| 8 | 6.00 |
| 9 | 4.24 |
| 10 | 6.90 |
| 13 | 5.90 |
| 14 | 8.10 |
| 15 | 1.99 |
| 17 | 56.42 |
| 18 | 124.53 |
| 19 | 117.75 |
| 20 | 15.21 |
| 21 | 21.23 |
| 22 | 1.96 |
| 24 | 1.99 |

According to the results in Table 2, it can be seen that the compounds of the present application can upregulate the expression level of miR-124 in PBMC.

<u>Pharmacological activity test: regulatory effect of quinoline derivatives on macrophage IL-6 and TNF-α secretion</u>

1. Test procedure

a. Induction of macrophages

Mouse bone marrow cells were isolated from the femur and tibia bone marrow of C75BL/6 mice of 6-8 week old raised under SPF conditions and inoculated in 6-well plates at a density of 1.5*$10^6$ cells/well/2 mL. 50 ng/mL M-CSF was added to each well and the cells were cultured in a $CO_2$ incubator for 3 days. On the third day, the culture medium was replaced with fresh medium containing 100 ng/mL M-CSF and the culture was continued for 3 days. On the 6th day, the supernatant was removed and the cells were washed once with DPBS. After adding 1 mL of DPBS, the macrophages were harvested using a cell scraper, centrifuged at 400 g for 10 min, and the supernatant was discarded. The cells were resuspended in fresh culture medium containing 50 ng/mL M-CSF, inoculated in a 96-well plate at a density of 2* $10^4$ cells/well/200 μL, and cultured in a $CO_2$ incubator overnight. 0, 3, 5, 10 μM of the test compound were added to each well (wherein well 0 contains 0.1% DMSO) respectively and incubate in a $CO_2$ incubator for 1 hour. Then 200 ng/ml LPS was added and the cell supernatant was collected after 6 hours of co-culture. ELISA kits were used to detect the secretion levels of IL-6 and TNF-α.

2. Test results

Some examples were selected for testing and compared with the test results of ABX-464. The test results are shown in Tables 3 and 4.

Table 3 Inhibition rate of IL-6 (compared to 0 μM concentration)

| Compound NO | Inhibition rate of IL-6 at 10 μM concentration |
|---|---|
| ABX-464 | 61% |
| 1 | 81% |
| 4 | 85% |
| 18 | 88% |

Table 4 Inhibition rate of TNFα (compared to 0 μM concentration)

| Compound NO | Inhibition rate of TNFα at 10 μM concentration |
|---|---|
| ABX-464 | 80% |
| 1 | 85% |
| 4 | 84% |
| 18 | 94% |

The results in Tables 3 and 4 show that the compounds of the present application can effectively inhibit the secretion of IL-6 and TNF-α by mouse macrophages, and achieve better inhibitory effects than the ABX-464 compound.

Pharmacological activity test: CYP enzyme inhibition studies

1. Test procedure

The inhibitors, probe substrates and reaction times of various enzyme subtypes are shown in Table 5. Commercially available mixed human liver microsomes were used, with the liver microsome concentrations of diclofenac, dextromethorphan, and midazolam being 0.1 mg/mL; and the liver microsome concentrations of phenacetin, bupropion, amodiaquine, mephenytoin, and testosterone being 0.2 mg/mL. Probe substrate, specific inhibitor and test compound (ABX464, compound 18) were diluted to the corresponding concentration of working solution for use. A mixed solution containing liver microsomes, $MgCl_2$, substrate, atopy inhibitor, and the test compound was prepared. 50 μL of 4 mM NADP solution was added to start the reaction, with the reaction time shown in the table. After the incubation, 600 μL of ice-cold ACN working solution containing IS was added to terminate the reaction, and the mixture was centrifuged and used for LC-MS/MS detection.

Table 5 inhibitors, probe substrates and reaction times of various enzyme subtypes

| CYP450 enzyme | Probe substrate (Final concentraion, μM) | Inhibitor (Final concentraion, μM) | Reaction time ( min) |
|---|---|---|---|
| CYP1A2 | Phenacetin (90) | α-naphthoflavone (0.2) | 20 |
| CYP2B6 | Bupropion (60) | Clopidogrel (1) | 10 |
| CYP2C8 | Amodiaquine (1) | Montelukast (4) | 5 |
| CYP2C9 | Diclofenac (8) | Sulfadifenazole (5) | 10 |
| CYP2C19 | S-Mephenytoin (20) | (+)-N-3-benzylnivanol (3) | 30 |
| CYP2D6 | Dextromethorphan (4) | Quinidine (2) | 10 |
| CYP3A4 | Midazolam (2) | Ketoconazole (1) | 5 |
| | Testosterone (50) | Ketoconazole (1) | 10 |

At different concentrations of the test compound or positive inhibitor, the percentage of remaining activity is determined by the ratio of the amount of characteristic metabolites of the probe substrate generated to the corresponding amount generated in the absence of the test compound or positive inhibitor. If a significant decrease in the amount of metabolite production is found at the highest concentration setting point, the half inhibitory concentration ($IC_{50}$) will be calculated using the log(inhibitor) vs. response -- Variable slope formula of GraphPad Prism software:

$$Y=Bottom + (Top - Bottom) / (1 + 10^{\wedge}((LogIC_{50} - X) * Hillslope))$$

X: Log (test compound or positive inhibitor concentration);
Y: Remaining activity percentage;
Top and Bottom: refer to the theoretical maximum and minimum remaining activity percentages respectively; and
Hillslope: slope coefficient or slope.

If there is no significant decrease in metabolite production at the highest concentration setting point (percentage of enzyme remaining activity > 50%), the half inhibitory concentration ($IC_{50}$) cannot be accurately calculated, and the reported $IC_{50}$ is greater than the highest concentration tested.

Text results

Table 6 Results of CYP enzyme inhibition studies of compounds

| CYP Enzyme subtype | ABX464 $IC_{50}$ (μM) | Compound 18 $IC_{50}$ (μM) |
|---|---|---|
| 1A2 | 0.097 | 21.6 |
| 2B6 | 86.7 | >100 |
| 2C8 | 35.9 | 40.4 |
| 2C9 | 15.1 | 20.2 |
| 2C19 | 4.92 | 5.69 |
| 2D6 | 17.2 | 48.0 |
| 3A4-Midazolam | 73 | >100 |
| 3A4-Testosterone | >100 | >100 |

The results in Table 6 show that compound 18 is significantly superior to ABX464 in terms of CYP1A2 inhibition.

<u>Pharmacological activity test: in vivo efficacy evaluation in mice</u>

1. Test procedure

In this experiment, male C57BL/6 mice (from Jiangsu Jicui Pharmaceutical Biotechnology Co., Ltd.) were used to establish a colitis model by freely drinking 3% DSS water. The grouping and dosing information are shown in the following table:

Table 7 Drug efficacy grouping and dosing information

| Group | Modeling | Dose (mg/kg) | Administration route | Frequency |
|---|---|---|---|---|
| G1- Normal control group | / | Vehicle (0.5%MC aqueous solution) | o.p. | QD |
| G2-DSS Enteritis model group | 8 days of 3%DSS water+3 days of water | Vehicle ( 0.5%MC aqueous solution) | o.p. | QD |
| G3-Compound 18 | 8 days of 3%DSS water +3 days of water | 10 | o.p. | QD |

(continued)

| Group | Modeling | Dose (mg/kg) | Administration route | Frequency |
|---|---|---|---|---|
| G4-ABX464 | 8 days of 3%DSS water +3 days of water | 40 | o.p. | QD |

After the adaptive feeding of mice, they were randomly divided into 4 groups according to their body weight: normal control group, DSS enteritis model group, compound 18-10 mg/kg group, and ABX464-40 mg/kg group. The day of grouping was recorded as D0. On D0-D8, mice in G2-G4 groups were given 3% DSS drinking water, which was changed every 3 days. On D9-D11, mice in G2-G4 groups were given normal drinking water. The corresponding solvents and drugs were intragastrically administered for 11 consecutive days from D0 to D11. From D0 to D11, mice in the G1 group received normal diet and water, and were given blank vehicle for 11 consecutive days. The mice were observed daily from D0 to D11, their weights were recorded, diarrhea scores and bloody stool scores were measured daily, and the DAI values were calculated. On D11, mice were euthanized, colon tissue was obtained, colon length was measured, colon weight was weighed, and photos were taken. The colon tissues were subjected to HE staining and pathological analysis.

Statistical analysis: Independent sample T test was used. #: $P<0.05$ compared with the normal control group; ##: $P<0.01$ compared with the normal control group; ###: $P<0.001$ compared with the normal control group. *: $P<0.05$ compared with the model control group; **: $P<0.01$ compared with the model control group; ***: $P<0.001$ compared with the model control group.

Text results

a. Body weight: The changes in body weight of mice in each group during the efficacy period are shown in Figure 1;
b. DAI score: The changes in DAI scores of mice in each group during the efficacy period are shown in Figure 2;
c. Colon length and colon weight.

Table 8 Colon length and colon weight of mice in each group

| Group | Colon length (cm) | Colon weight (g) |
|---|---|---|
| G1- Normal control group | 7.3 | 0.3020 |
| G2-DSS Enteritis model group | 5.7### | 0.2833 |
| G3- Compound 18-10 mg/kg | 6.6*** | 0.3710** |
| G4-ABX464-40 mg/kg | 6.7* | 0.3776* |

d. Pathological score:

Table 9 Pathological scores of mice in each group

| Group | Pathological score |
|---|---|
| G1- Normal control group | 0 |
| G2-DSS Enteritis model group | 7.25### |
| G3- Compound 18-10 mg/kg | 4.00** |
| G4-ABX464-40 mg/kg | 3.38** |

e. Conclusion:

Compared with the normal control group, the body weight of mice in the DSS enteritis model group was significantly reduced, the DAI score was significantly increased, the colon length of mice was significantly reduced, and the pathological score was significantly increased, indicating that the enteritis model was successfully established. Compared with the DSS enteritis model control group, the body weight of mice in the compound 18-10 mg/kg group was significantly increased; the DAI scores of the compound 18-10 mg/kg group and the ABX464-40 mg/kg group were significantly reduced; the colon length and colon weight of mice in the compound 18-10 mg/kg group and the ABX464-40 mg/kg group were significantly increased; the colon pathological score of mice in the compound 18-10 mg/kg group and the ABX464-40 mg/kg group was significantly reduced. Those suggest that both compound 18 and ABX464 can significantly reduce

enteritis disease and pathological scores, reduce intestinal shortening caused by enteritis, and increase intestinal weight.

## Claims

**1.** A quinoline derivative compound or a pharmaceutically acceptable salt thereof, having the structural formula as shown in Formula I:

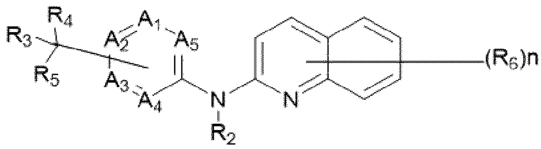

I

wherein:

$A_1$, $A_2$, $A_3$, $A_4$ and $A_5$ are each independently selected from N or C-$R_1$, wherein $A_1$, $A_2$, $A_3$, $A_4$ and $A_5$ comprise no more than 2 N;
$R_1$ is selected from hydrogen, deuterium, halogen, cyano, amino, hydroxyl, nitro, $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl, $C_1$-$C_3$ alkoxy and phosphate; wherein the $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, $C_1$-$C_3$ alkoxy are each independently substituted with one or more same or different substituents selected from $C_1$-$C_3$ alkyl, $C_1$-$C_6$ alkoxy, $C_3$-$C_7$ cycloalkyl, $C_3$-$C_6$ heterocycloalkyl, deuterium, halogen, cyano, amino, hydroxyl and nitro;
$R_2$ is selected from hydrogen, alkyl, cycloalkyl and heterocycloalkyl; wherein the alkyl, cycloalkyl and heterocycloalkyl are each independently substituted with one or more same or different substituents selected from halogen, hydroxyl, carboxyl, ester group, alkyl, alkoxy, deuterium, haloalkyl, haloalkoxy, nitro, amino and cyano;
$R_3$ and $R_4$ are each independently selected from cyano, hydroxyl, $C_1$-$C_3$ alkyl, $C_1$-$C_3$ alkanoyl, $C_1$-$C_3$ alkylamino, and $C_1$-$C_3$ alkoxy; wherein the $C_1$-$C_3$ alkyl, $C_1$-$C_3$ alkanoyl, $C_1$-$C_3$ alkylamino, and $C_1$-$C_3$ alkoxy are each independently substituted with one or more same or different substituents selected from halogen, hydroxyl, carboxyl, ester group, alkyl, alkoxy, deuterium, haloalkyl, haloalkoxy, nitro, amino and cyano;
or $R_3$ and $R_4$ are connected to form $C_3$-$C_6$ cycloalkyl or $C_3$-$C_6$ heterocycloalkyl;
$R_5$ is selected from deuterium, halogen, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;
$R_3$, $R_4$ and $R_5$ are not all alkyl or cycloalkyl;
these $R_6$ are the same or different, and are each independently selected from hydrogen, deuterium, halogen, cyano, hydroxyl$C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl, phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro; and
n=1, 2 or 3.

**2.** The quinoline derivative compound or a pharmaceutically acceptable salt thereof according to claim 1, wherein the structural formula of the quinoline derivative compound is shown in Formula I-1:

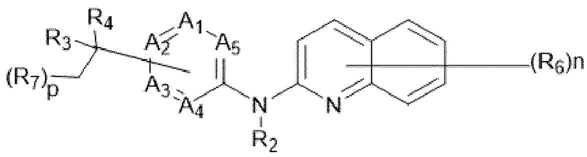

I-1

wherein:

these $R_7$ are the same or different, and are each independently selected from hydrogen, halogen, amino, hydroxyl, nitro, $C_1$-$C_3$ alkyl, $C_1$-$C_3$haloalkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl and $C_1$-$C_3$ alkoxy;
p=1, 2 or 3; and
$A_1$, $A_2$, $A_3$, $A_4$, $A_5$, $R_2$, $R_3$, $R_4$, and $R_6$ are as defined in claim 1.

3. The quinoline derivative compound or a pharmaceutically acceptable salt thereof according to claim 1, wherein the structural formula of the quinoline derivative compound is shown in Formula I-2:

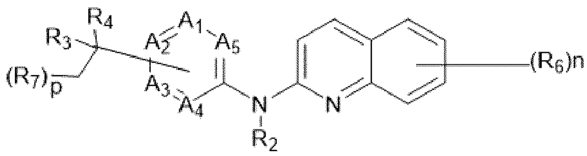

I-2.

4. The quinoline derivative compound or a pharmaceutically acceptable salt thereof according to claim 1, wherein the $R_2$ is hydrogen or $C_3$-$C_8$ heterocycloalkyl; wherein the $C_3$-$C_8$ heterocycloalkyl is optionally substituted with one or more same or different substituents in hydroxyl and carboxyl.

5. The quinoline derivative compound or a pharmaceutically acceptable salt thereof according to claim 1, wherein the structural formula of the quinoline derivative compound is shown in Formulae I-3, I-4 or I-5:

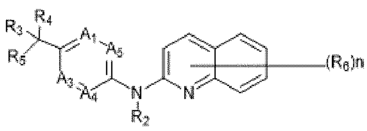 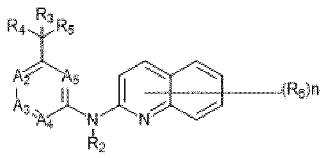 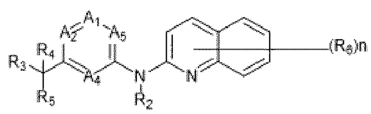

I-3 I-4 I-5.

6. The quinoline derivative compound or a pharmaceutically acceptable salt thereof according to claim 5, wherein the $R_3$ and $R_4$ are connected to form $C_3$-$C_6$ cycloalkyl or $C_3$-$C_6$ heterocycloalkyl, and together with $R_5$ to form a group of

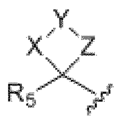;

wherein, X, Y and Z are each independently selected from $(CH_2)_m$, NH or O; and
m=0, 1 or 2.

7. The quinoline derivative compound or a pharmaceutically acceptable salt thereof according to claim 6, wherein the $R_3$

and $R_4$ are connected to form $C_3$-$C_6$ cycloalkyl or $C_3$-$C_6$ heterocycloalkyl, and together with $R_5$ to form a group as below:

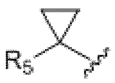, 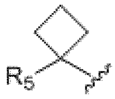, 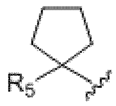, 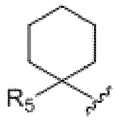, 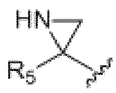, 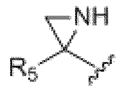, 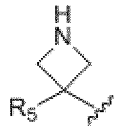,

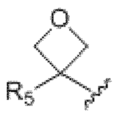, 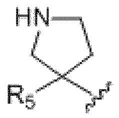, 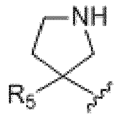, 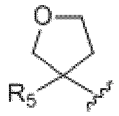, 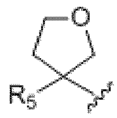, 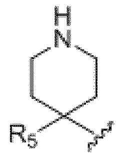 or 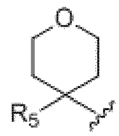.

**8.** The quinoline derivative compound or a pharmaceutically acceptable salt thereof according to claim 5, wherein the structural formula of the quinoline derivative compound is shown in Formulae I-7 or I-8:

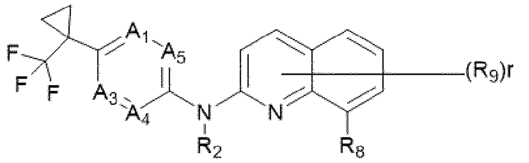

I-7

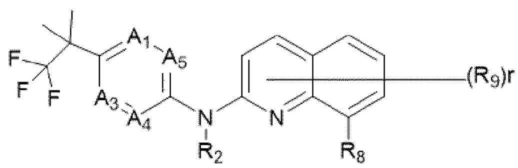

I-8

wherein:

$R_8$ is selected from deuterium, halogen, cyano, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;

these $R_9$ are the same or different, and are each independently selected from hydrogen, deuterium, halogen, cyano, hydroxyl, $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl, phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro;

r=0, 1 or 2; and

$A_1$, $A_2$, $A_3$, $A_4$, $A_5$, $R_2$, $R_5$, and $R_6$ are as defined in claim 1.

**9.** The quinoline derivative compound or a pharmaceutically acceptable salt thereof according to claim 8, wherein the $R_2$ is selected from hydrogen,

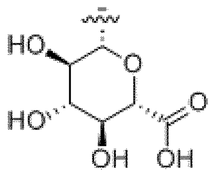 or 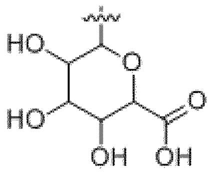.

**10.** The quinoline derivative compound or a pharmaceutically acceptable salt thereof according to any one of claims 1-9, wherein the quinoline derivative compound is selected from any of the following compounds:

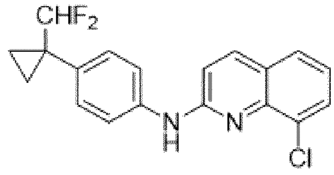
1

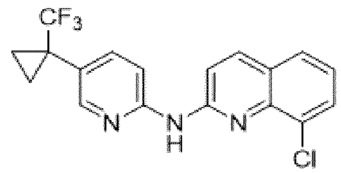
2

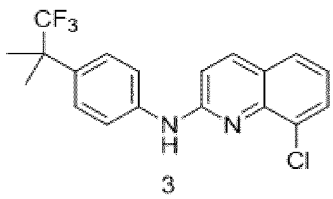
3

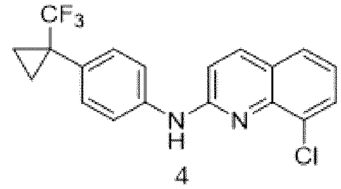
4

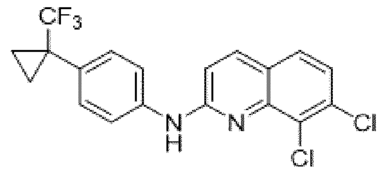
5

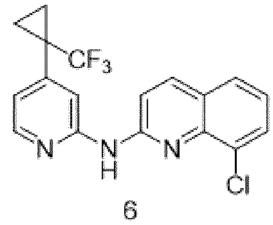
6

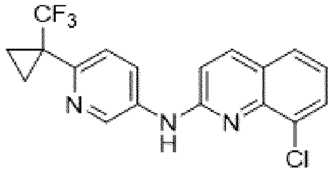
7

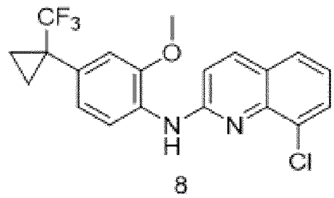
8

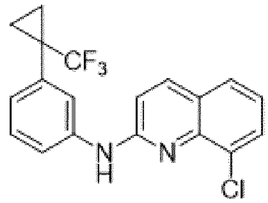
9

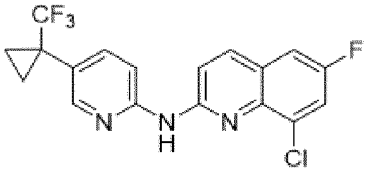
10

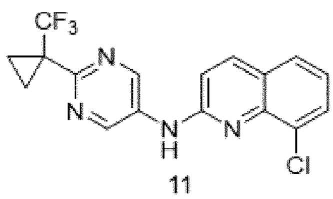
11

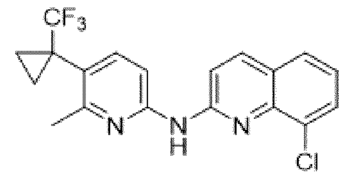
12

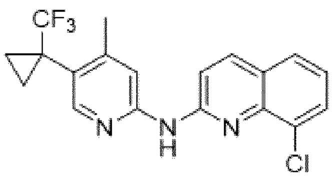
13

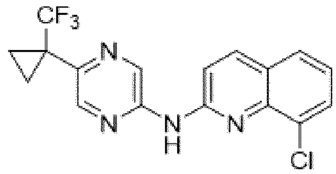
14

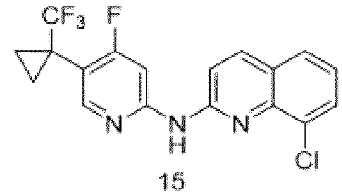
15

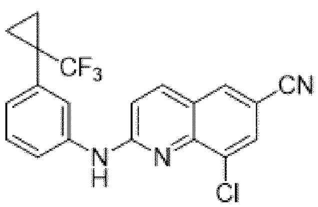
16

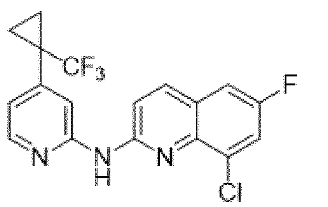
17

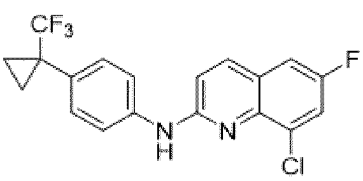
18

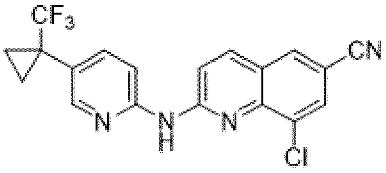

19

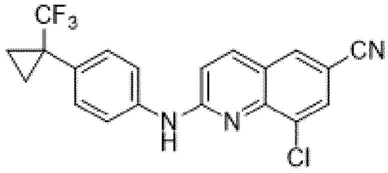

20

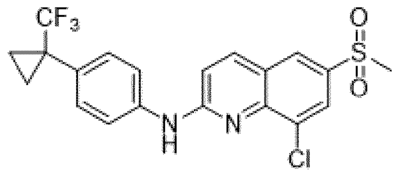

21

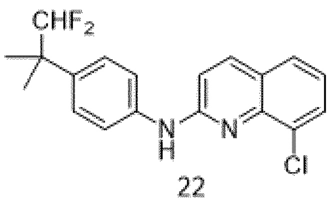

22

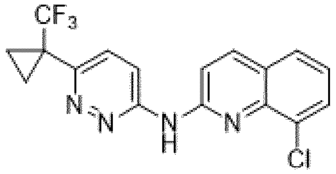

23

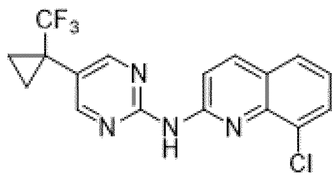

24

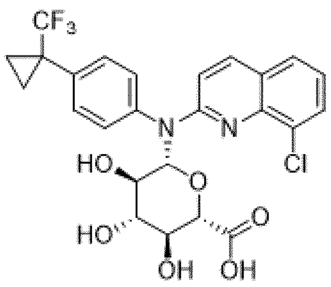

25

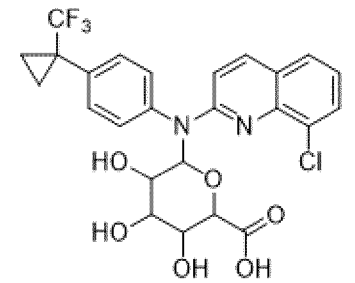

26

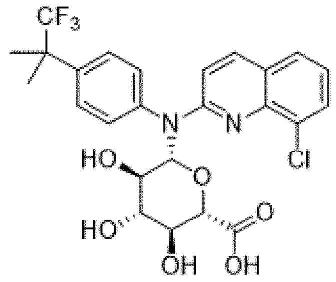

27

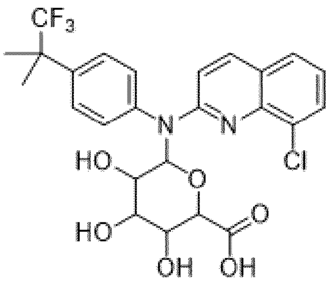

28

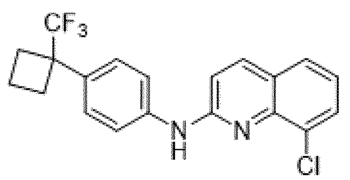

29

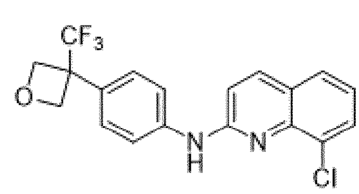

30

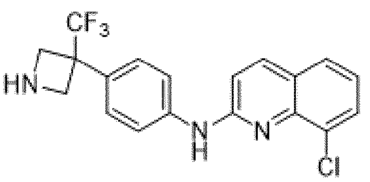

31

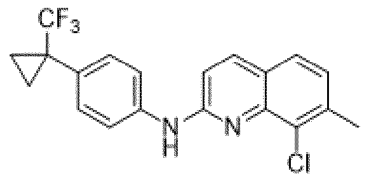

32

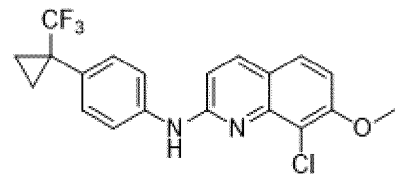

33

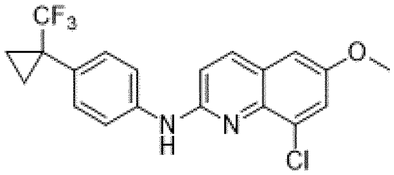

34

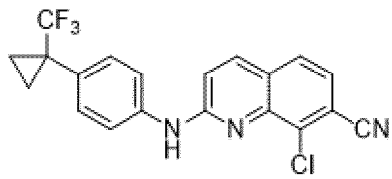

35

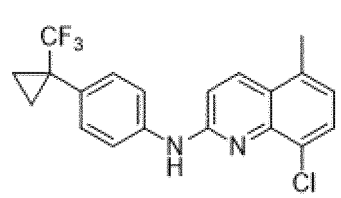

36

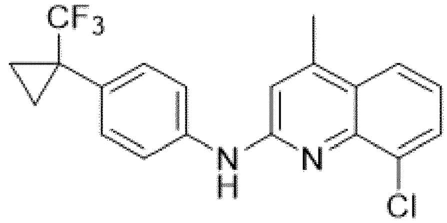

37

.

**11.** A method for preparing a quinoline derivative compound or a pharmaceutically acceptable salt thereof according to claim 1, comprising:

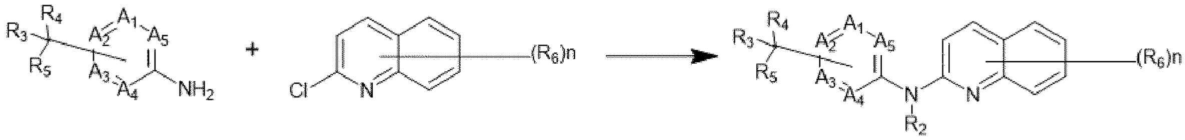

wherein:

$A_1$, $A_2$, $A_3$, $A_4$ and $A_5$ are each independently selected from N or C-$R_1$, wherein $A_1$, $A_2$, $A_3$, $A_4$ and $A_5$ comprise no more than 2 N;

$R_1$ is selected from hydrogen, deuterium, halogen, cyano, amino, hydroxyl, nitro, $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, aminosulfonyl, $C_1$-$C_3$ alkoxy and phosphate; wherein the $C_1$-$C_3$ alkyl, $C_1$-$C_3$ alkanoyl, $C_1$-$C_3$ alkylsulfonyl, $C_1$-$C_3$ alkylamino, $C_1$-$C_3$ alkylphosphate, $C_1$-$C_3$ alkylaminosulfonyl, $C_1$-$C_3$ alkoxy are each independently substituted with one or more same or different substituents selected from $C_1$-$C_3$ alkyl, $C_1$-$C_6$ alkoxy, $C_3$-$C_7$ cycloalkyl, $C_3$-$C_6$ heterocycloalkyl, deuterium, halogen, cyano, amino, hydroxyl and nitro;

$R_2$ is selected from hydrogen, alkyl, cycloalkyl and heterocycloalkyl; wherein the alkyl, cycloalkyl and heterocycloalkyl are each independently substituted with one or more same or different substituents selected from halogen, hydroxyl, carboxyl, alkyl, alkoxy, deuterium, haloalkyl, haloalkoxy, nitro, amino and cyano;

$R_3$ and $R_4$ are each independently selected from cyano, hydroxyl, $C_1$-$C_3$ alkyl, $C_1$-$C_3$ alkanoyl, $C_1$-$C_3$ alkylamino, and $C_1$-$C_3$ alkoxy;

or $R_3$ and $R_4$ are connected to form $C_3$-$C_6$ cycloalkyl or $C_3$-$C_6$ heterocycloalkyl;

$R_5$ is selected from deuterium, halogen, amino, hydroxyl, nitro, $C_1$-$C_6$ alkyl, $C_1$-$C_6$haloalkyl, $C_1$-$C_6$ alkanoyl, $C_1$-$C_6$ alkylsulfonyl, $C_1$-$C_6$ alkylamino, $C_1$-$C_6$ alkylphosphate, $C_1$-$C_6$ alkylaminosulfonyl, aminosulfonyl and $C_1$-$C_6$ alkoxy;

$R_3$, $R_4$ and $R_5$ are not all alkyl or cycloalkyl;

these $R_6$ are the same or different, and are each independently selected from hydrogen, deuterium, halogen, cyano, hydroxyl$C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, aminosulfonyl, $C_3$-$C_7$ cycloalkyl, $C_3$-$C_6$ heterocycloalkyl, $C_2$-$C_6$ alkenyl, $C_2$-$C_6$ alkynyl, phosphate; wherein the $C_1$-$C_3$ alkyl, $C_1$-$C_3$ alkoxy, $C_1$-$C_3$ alkanoyl, $C_1$-$C_3$ alkylamino, $C_1$-$C_3$ alkylsulfonyl, $C_1$-$C_3$ alkylphosphate, $C_1$-$C_3$ alkylaminosulfonyl, $C_3$-$C_7$ cycloalkyl, $C_3$-$C_6$ heterocycloalkyl, $C_2$-$C_6$ alkenyl, $C_2$-$C_6$ alkynyl are each independently and optionally substituted with one or more same or different substituents selected from $C_1$-$C_6$ alkyl, $C_1$-$C_6$ alkoxy, $C_3$-$C_7$ cycloalkyl, $C_3$-$C_6$ heterocycloalkyl, deuterium, halogen, cyano, amino, hydroxyl, and nitro; and

n=1, 2 or 3.

**12.** A pharmaceutical composition comprising the compound or a pharmaceutically acceptable salt thereof according to any one of claims 1 to 10, and one or more pharmaceutically acceptable carriers, diluents or excipients.

**13.** Use of the compound or a pharmaceutically acceptable salt thereof according to any one of claims 1 to 10 in the preparation of a drug for preventing and/or treating an inflammatory disease or cancer.

**14.** The use according to claim 13, wherein the inflammatory disease is selected from autoimmune-related inflammatory diseases, inflammatory diseases in the central nervous system (CNS), inflammatory diseases in joints, inflammatory diseases in the digestive tract, inflammatory diseases in the skin, other inflammatory diseases related to epithelial

cells, inflammation related to cancer, inflammation related to irritation, and inflammation related to injury.

**15.** The use according to claim 13, wherein the inflammatory disease is selected from inflammatory bowel disease, rheumatoid arthritis, multiple sclerosis, Alzheimer's disease, Parkinson's disease, osteoarthritis, atherosclerosis, ankylosing spondylitis, psoriasis, dermatitis, systemic lupus erythematosus, Sjogren's syndrome, bronchitis, asthma, and inflammation related to colon cancer.

**16.** The use according to claim 15, wherein the inflammatory bowel disease is ulcerative colitis (UC) or Crohn's disease (CD).

**17.** The use according to claim 13, wherein the cancer is selected from leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous gland carcinoma, papillary carcinoma, cystadenocarcinoma, medullary carcinoma, bronchogenic carcinoma, hepatocarcinoma, bile duct carcinoma, choriocarcinoma, seminoma, embryonic carcinoma, Wilm's tumor, cervical cancer, uterine cancer, testicular cancer, lung cancer, bladder cancer, glioma, medulloblastoma, craniopharyngioma, ependymoma, pineal tumor, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumors, urothelial carcinoma, ureteric tumors, thyroid cancer, osteoma, neuroblastoma, brain tumors, and myeloma.

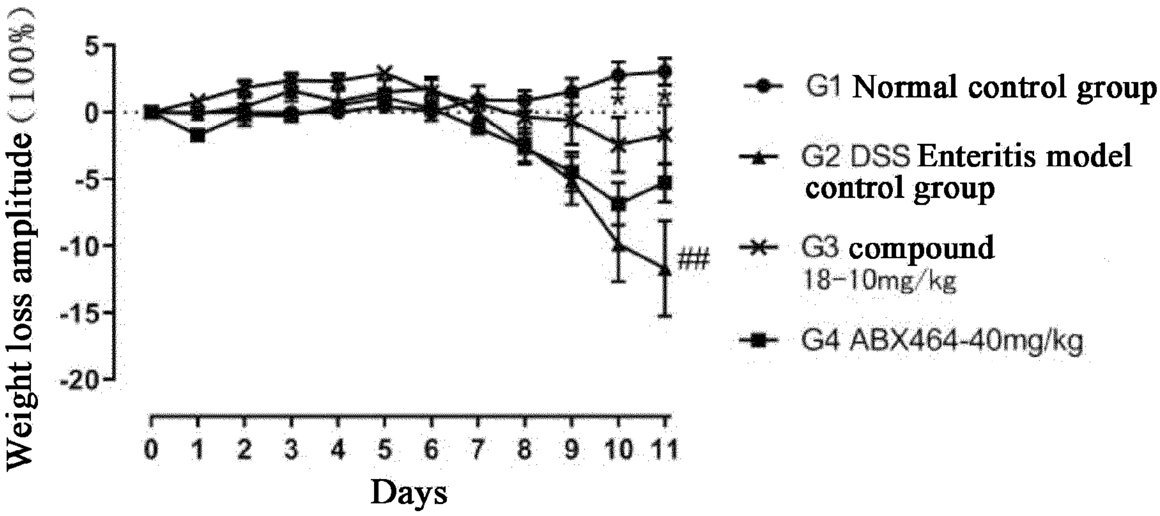
Weight loss amplitude（100%）
5
0
-5
-10
-15
-20
0 1 2 3 4 5 6 7 8 9 10 11
Days
##
G1 Normal control group
G2 DSS Enteritis model control group
G3 compound 18-10mg/kg
G4 ABX464-40mg/kg

FIG. 1

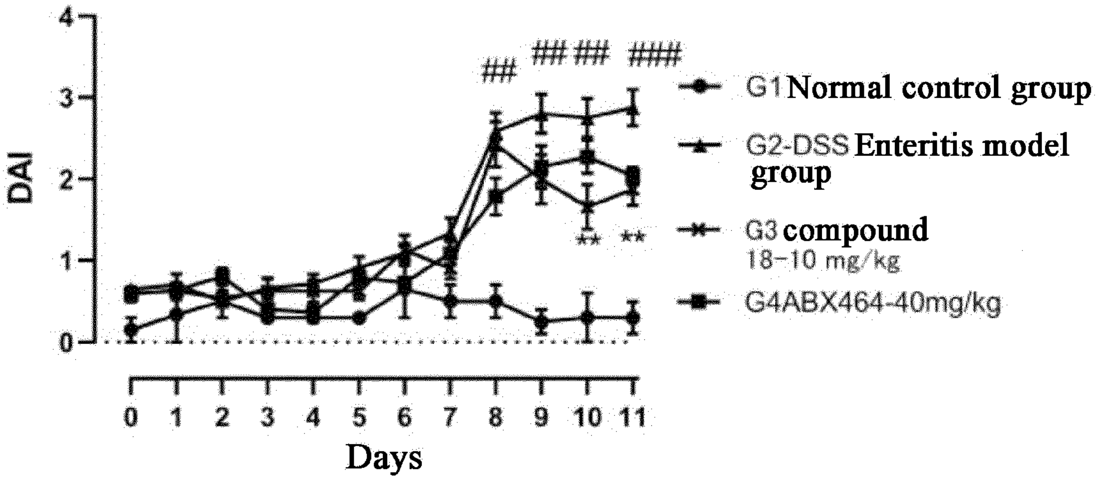
DAI
4
3
2
1
0
0 1 2 3 4 5 6 7 8 9 10 11
Days
##
## ##
###
**
**
G1Normal control group
G2-DSS Enteritis model group
G3 compound 18-10 mg/kg
G4ABX464-40mg/kg

FIG. 2

**INTERNATIONAL SEARCH REPORT**

International application No. **PCT/CN2023/134615**

**A. CLASSIFICATION OF SUBJECT MATTER**

C07D 215/38(2006.01)i； C07D 405/12(2006.01)i； C07D 401/12(2006.01)i； C07D 221/16(2006.01)i； A61P 29/00(2006.01)i； A61P 25/00(2006.01)i； A61P 35/00(2006.01)i； A61K 31/47(2006.01)i； A61K 31/473(2006.01)i

According to International Patent Classification (IPC) or to both national classification and IPC

**B. FIELDS SEARCHED**

Minimum documentation searched (classification system followed by classification symbols)

IPC： C07D， A61P， A61K

Documentation searched other than minimum documentation to the extent that such documents are included in the fields searched

Electronic data base consulted during the international search (name of data base and, where practicable, search terms used)

CNTXT, VEN, DWPI, ENTXTC, CNKI, 万方, WANFANG, 百度学术, BAIDU SCHOLAR, ISI web of Science, STN; 柯菲平医药, 秦引林, 苏梅, 王超磊, 张亚, 喹啉, 炎性疾病, 炎症, miR-124, 促炎细胞因子, IL-6, TNF-α, quinoline, microRNA, inflammation, cancer, tumor, 根据式I的结构检索, structural search according to formula I.

**C. DOCUMENTS CONSIDERED TO BE RELEVANT**

| Category* | Citation of document, with indication, where appropriate, of the relevant passages | Relevant to claim No. |
|---|---|---|
| X | CN 113646431 A (ABIVAX et al.) 12 November 2021 (2021-11-12)<br>claims 1-27, and description, paragraphs 3, 94-245, 252-272, 274-279, 308-336, and 367-379 | 1-17 |
| X | CN 115433127 A (JIANGSU HENGRUI MEDICINE CO., LTD. et al.) 06 December 2022 (2022-12-06)<br>abstract of description, claims 1-25, and description, embodiments 1-5, and paragraphs 461-525 | 1-17 |
| X | CN 107207463 A (ABIVAX et al.) 26 September 2017 (2017-09-26)<br>claims 1-15, and description, embodiments 1-9 | 1-17 |
| X | CN 113825509 A (ABIVAX et al.) 21 December 2021 (2021-12-21)<br>abstract of description, claims 1-17, and description, tables 1-2 | 1-17 |
| A | US 2017197938 A1 (ABIVAX et al.) 13 July 2017 (2017-07-13)<br>entire document | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **29 January 2024** | **01 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
**PCT/CN2023/134615**

| C. DOCUMENTS CONSIDERED TO BE RELEVANT | | |
|---|---|---|
| Category* | Citation of document, with indication, where appropriate, of the relevant passages | Relevant to claim No. |
| A | CN 115605205 A (ABIVAX) 13 January 2023 (2023-01-13)<br>entire document | 1-17 |

Form PCT/ISA/210 (second sheet) (July 2022)

**INTERNATIONAL SEARCH REPORT**
**Information on patent family members**

International application No.
**PCT/CN2023/134615**

| Patent document cited in search report | Publication date (day/month/year) | Patent family member(s) | Publication date (day/month/year) |
|---|---|---|---|
| CN 113646431 A | 12 November 2021 | EP 3670659 A1 | 24 June 2020 |
| | | CA 3122644 A1 | 25 June 2020 |
| | | KR 20210137989 A | 18 November 2021 |
| | | JP 2022513515 A | 08 February 2022 |
| | | MX 2021007116 A | 11 August 2021 |
| | | US 2022145406 A1 | 12 May 2022 |
| | | EP 3898973 A1 | 27 October 2021 |
| | | WO 2020127853 A1 | 25 June 2020 |
| | | AU 2019408250 A1 | 08 July 2021 |
| CN 115433127 A | 06 December 2022 | None | |
| CN 107207463 A | 26 September 2017 | CA 3166018 A1 | 21 January 2016 |
| | | DK 3169328 T3 | 30 May 2022 |
| | | PL 3169328 T3 | 11 July 2022 |
| | | JP 2017522313 A | 10 August 2017 |
| | | JP 6884690 B2 | 09 June 2021 |
| | | BR 112017000682 A2 | 09 January 2018 |
| | | RU 2021134325 A | 02 December 2021 |
| | | EP 3169328 A2 | 24 May 2017 |
| | | EP 3169328 B1 | 09 March 2022 |
| | | AU 2021201960 A1 | 29 April 2021 |
| | | AU 2021201960 B2 | 23 February 2023 |
| | | US 2021047273 A1 | 18 February 2021 |
| | | US 11649210 B2 | 16 May 2023 |
| | | MX 2021006754 A | 15 July 2021 |
| | | KR 20230078827 A | 02 June 2023 |
| | | JP 2021038219 A | 11 March 2021 |
| | | JP 7208957 B2 | 19 January 2023 |
| | | SI 3169328 T1 | 30 November 2022 |
| | | WO 2016009065 A2 | 21 January 2016 |
| | | AU 2023202386 A1 | 29 June 2023 |
| | | HUE 058713 T2 | 28 September 2022 |
| | | CU 20170004 A7 | 05 June 2017 |
| | | CU 24459 B1 | 03 December 2019 |
| | | US 2022315535 A1 | 06 October 2022 |
| | | US 2021087145 A1 | 25 March 2021 |
| | | US 11649211 B2 | 16 May 2023 |
| | | EP 2974729 A1 | 20 January 2016 |
| | | US 2017204063 A1 | 20 July 2017 |
| | | US 10435370 B2 | 08 October 2019 |
| | | KR 20170032361 A | 22 March 2017 |
| | | KR 102537958 B1 | 30 May 2023 |
| | | LT 3169328 T | 10 June 2022 |
| | | HRP 20220598 T1 | 24 June 2022 |
| | | MX 2017000533 A | 27 April 2017 |
| | | PT 3169328 T | 27 May 2022 |
| | | RU 2017101407 A | 17 August 2018 |
| | | RU 2017101407 A3 | 17 January 2019 |
| | | RU 2760686 C2 | 29 November 2021 |
| | | EP 3998070 A1 | 18 May 2022 |
| | | CU 20190043 A7 | 03 December 2019 |

Form PCT/ISA/210 (patent family annex) (July 2022)

**INTERNATIONAL SEARCH REPORT**
**Information on patent family members**

International application No.
**PCT/CN2023/134615**

| Patent document cited in search report | Publication date (day/month/year) | Patent family member(s) | Publication date (day/month/year) |
|---|---|---|---|
| | | CU 24565 B1 | 13 January 2022 |
| | | JP 2023052265 A | 11 April 2023 |
| | | AU 2015289033 A1 | 02 February 2017 |
| | | ES 2914066 T3 | 07 June 2022 |
| | | CA 2954951 A1 | 21 January 2016 |
| | | CA 2954951 C | 18 October 2022 |
| | | AU 2020200333 A1 | 06 February 2020 |
| | | AU 2020200333 B2 | 21 January 2021 |
| | | RS 63231 B1 | 30 June 2022 |
| | | US 2019382347 A1 | 19 December 2019 |
| | | US 10981874 B2 | 20 April 2021 |
| CN 113825509 A | 21 December 2021 | JP 2022513516 A | 08 February 2022 |
| | | EP 3669873 A1 | 24 June 2020 |
| | | CA 3122900 A1 | 25 June 2020 |
| | | KR 20210136970 A | 17 November 2021 |
| | | US 2022023324 A1 | 27 January 2022 |
| | | WO 2020127843 A1 | 25 June 2020 |
| | | MA 54515 A | 27 October 2021 |
| | | BR 112021012100 A2 | 08 September 2021 |
| | | IL 284125 A | 31 August 2021 |
| | | MX 2021007114 A | 11 August 2021 |
| | | AU 2019410432 A1 | 15 July 2021 |
| | | EP 3897640 A1 | 27 October 2021 |
| US 2017197938 A1 | 13 July 2017 | EP 3169675 A1 | 24 May 2017 |
| | | EP 3169675 B1 | 19 September 2018 |
| | | EP 2975034 A1 | 20 January 2016 |
| | | WO 2016009066 A1 | 21 January 2016 |
| | | ES 2701922 T3 | 26 February 2019 |
| CN 115605205 A | 13 January 2023 | IL 296522 A | 01 November 2022 |
| | | AU 2021238792 A1 | 13 October 2022 |
| | | KR 20220152292 A | 15 November 2022 |
| | | CA 3172179 A1 | 23 September 2021 |
| | | EP 4121053 A1 | 25 January 2023 |
| | | US 2023142547 A1 | 11 May 2023 |
| | | WO 2021186053 A1 | 23 September 2021 |
| | | JP 2023521564 A | 25 May 2023 |
| | | MX 2022011596 A | 18 October 2022 |
| | | BR 112022018793 A2 | 29 November 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310062976
- WO 2015001518 A1


### Non-patent literature cited in the description


- **KOUKOS et al.** *Gastroenterology*, 2013, vol. 145 (4), 842-52